# EUROPEAN PATENT APPLICATION

(11) **EP 1 191 105 A1**
(43) Date of publication of application: **27.03.2002**
(21) Application number: 00203375.1
(22) Date of filing: 25.09.2000
(51) Int. Cl.: C12N 15/861, C12N 7/01, A61K 48/00

(54) **Gene delivery vectors provided with a tissue tropism for T-lymphocytes**

(71) Applicant: Galapagos Genomics B.V., 2301 CA Leiden (NL); Introgene B.V., 2301 CA Leiden (NL)
(72) Inventor: van Zutphen, Marlijn, 2352 XL Leiderdorp (NL); van Es, Helmith Hendrikus Gerardus, 2133 DJ Hoofddorp (NL); Havenga, Menzo Jans Emco, 2401 KG Alphen a/d Rijn (NL)
(74) Representative: Prins, Hendrik Willem

(57) **Abstract**

It is an object of the invention to provide a viral vector that has improved tropism for T-lymphocytes, such that said viral vector is more capable/efficient in providing a desired nucleotide sequence to a T-lymphocyte.

This object is achieved by providing a (chimeric) virus or virus particle that is suitable for use as a viral vector, and that has been provided with an altered/modified viral coat that confers increased tropism for T-lymphocytes upon said virus particle.

## Description

### Field of the invention

The invention relates to the field of molecular genetics and medicine. In particular the present invention relates to the field of functional genomics and gene therapy, more in particular to functional genomics using adenoviruses.

### Background of the invention

In functional genomics, genetic information with unknown function but somehow pre-selected for, is usually delivered to a host cell in order to either correct (supplement) a genetic deficiency in said cell, or to inhibit an undesired function in said cell or to otherwise induce a phenotype. Of course the genetic information can also be intended to provide the host cell with a desired function, e.g. to supply a secreted protein or express a transcription factor.

Many different methods have been developed to introduce new genetic information into cells. Although many different systems may work on cell-lines cultured *in vitro*, only the group of viral vector mediated gene delivery methods seems to be able to meet the required efficiency of gene transfer *in vivo*. Thus for gene therapy purposes most of the attention is directed towards the development of suitable viral vectors. Today, most of the attention for the development of suitable viral vectors is directed towards those vectors that are based on adenoviruses. Studies in clinical trials have provided valuable information on the use of these vectors in patients. Moreover adenoviral vectors are relatively easy to concentrate and purify. For functional genomics adenoviral vectors are also ideally suited. They can be used to build gene expression libraries that can be used with specific cell based assays to search for genes or antagonists of those genes that give a desired phenotype. They can also be used to validate genes further that have been isolated using other gene selection techniques such as comparative expression profiling and subtraction techniques. Validation using adenoviral vectors can be done *in vitro* as well as *in vivo* using either in situ or *in vitro* cell or tissue based assays or appropriate animal models.

Some characteristics of the current adenoviral vectors limit their use in specific applications. For instance endothelial cells, smooth muscle cells and T-lymphocytes are not easily transduced by the current generation of adenoviral vectors. For many gene therapy or functional genomics applications, preferably these types of cells should be genetically modified. Disease areas for which efficient gene transfer into these cell types is desirable include but are not limited to autoimmune disorders, cancer, infectious diseases, cardiovascular diseases and bone disorders.

The present invention was made in the course of the manipulation of adenoviral vectors to obtain efficient gene transfer into T-lymphocytes in particular human T-lymphocytes. Therefor in the following section a brief introduction of T-lymphocytes and adenoviruses is given.

### T-lymphocytes

T-lymphocytes are formed in the bone marrow, migrate to and mature in the thymus and then enter the peripheral blood and lymphatic circulation. T-lymphocytes are subdivided into three distinct types of cells: helper T-lymphocytes, suppressor T-lymphocytes, and cytotoxic T-lymphocytes. T-lymphocytes, unlike B-lymphocytes, do not produce antibody molecules, but express a heterodimeric cell surface receptor that recognizes peptide fragments of antigenic proteins that are attached to proteins of the major histocompatibility complex (MHC) and expressed on the surfaces of target T-lymphocytes (e.g. Abbas *et al*, 1991).

Human cytotoxic T-lymphocytes (CTLs) are typically of the CD3⁺, CD8⁺, CD4⁻ phenotype and lyse cells that display fragments of foreign antigens associated with MHC class I molecules on their cell surfaces. Target T-lymphocytes for CTL recognition include normal cells expressing antigens after infection by viruses or other pathogens; and tumor cells that have undergone transformation and are expressing mutated proteins or are over-expressing normal proteins.

Helper T-lymphocytes are also CD3⁺ but can be distinguished from cytotoxic T-lymphocytes by expression of CD4 but absence of the CD8 membrane protein. CD4⁺ helper T-lymphocytes recognize fragments of antigens presented in association with MHC class II molecules, and primarily function to produce cytokines that amplify antigen-specific T- and B-cell responses and activate accessory immune cells such as monocytes or macrophages (e.g. Abbas *et al*, 1991).

CD4⁺ helper and CD8⁺ cytotoxic T-lymphocytes are important components of the host immune response to viruses, bacterial pathogens and tumors. As a result, individuals with congenital, acquired or iatrogenic T-cell immunodeficiency diseases may develop life threatening infections or malignancies (for example, SCID, AIDS, etc.). Persons with diseases that are related to a deficiency of immunologically competent T-lymphocytes can potentially have specific immunity restored through adoptive immunotherapy, alternatively called adoptive transfer. In adoptive immunotherapy, one or more specific immunities can be conferred upon an individual by transferring T-lymphocytes having the desired antigenic specificities. The cells of interest may be derived from the immunodeficient host or from a compatible specifically immunized host. The latter source is of course especially important in situations in which the immunodeficient host has an insufficient number of T-lymphocytes, or has T-lymphocytes that are insufficiently effective. Efficient and reproducible gene transfer into T-lymphocytes, in particular human T-lymphocytes, is of prime importance in the validation of new genes, and for the development of new immuno or gene therapies.

T-lymphocytes can be isolated or enriched for by using cell immuno affinity methods based for example on magnetic beads having anti CD3, CD4 or CD8 antibodies on their surface, thus cell isolation is based on T-cell specific cell surface markers. Common methodology used is the technology developed by Miltenyi *et al*. Preferred is to use the technology for depletion of cell types other than T-lymphocytes so that activation of the resting T-cells by for example CD3 antibodies is avoided. This is done by using antibodies against markers for other cell types of the hemopoietic system such as monocytes and B-lymphocytes.

### Adenoviruses

Adenoviruses contain a linear double-stranded DNA molecule of approximately 36000 base pairs. It contains identical Inverted Terminal Repeats (ITR) of approximately 90-140 base pairs with the exact length depending on the serotype. The viral origins of replication are within the ITRs exactly at the genome ends. The transcription units are divided in early and late regions. Shortly after infection the E1A and E1B proteins are expressed and function in transactivation of cellular and adenoviral genes. The early regions E2A and E2B encode proteins (DNA binding protein, pre-terminal protein and polymerase) required for the replication of the adenoviral genome (reviewed in van der Vliet, 1995). The early region E4 encodes several proteins with pleiotropic functions e.g. transactivation of the E2 early promoter, facilitating transport and accumulation of viral mRNAs in the late phase of infection and increasing nuclear stability of major late pre-mRNAs (reviewed in Leppard, 1997). The early region 3 encodes proteins that are involved in modulation of the immune response of the host (Wold *et al*, 1995). The late region is transcribed from one single promoter (major late promoter) and is activated at the onset of DNA replication. Complex splicing and poly-adenylation mechanisms give rise to more than 12 RNA species coding for core proteins, capsid proteins (penton, hexon, fiber and associated proteins), viral protease and proteins necessary for the assembly of the capsid and shut-down of host protein translation (Imperiale *et al*, 1995).

### Interaction between virus and host cell

The interaction of the virus with the host cell has mainly been investigated with the serotype C viruses Ad2 and Ad5. Binding occurs via interaction of the knob region of the protruding fiber with a cellular receptor. A receptor for Ad2, Ad5 and probably more adenoviruses, is known as the 'Coxsackievirus and Adenovirus Receptor' or CAR protein (Bergelson *et al*, 1997). Internalization is mediated through interaction of the RGD (Arg,Gly,Asp) sequence present in the penton base with cellular β1-integrins (Wickham *et al*, 1993). This may not be true for all serotypes, for example serotype 40 and 41 do not contain a RGD sequence in their penton base sequence (Kidd *et al*, 1993).

### The fiber protein

The initial step for successful infection is binding of adenovirus to its target cell, a process mediated through the fiber protein. The fiber protein has a trimeric structure (Stouten *et al*, 1992) with different lengths depending on the virus serotype (Signas *et al*, 1985; Kidd *et al*, 1993). Different serotypes have polypeptides with structurally similar N- and C-termini, but different middle stem regions. The first 30 amino acids at the N-terminus are involved in anchoring of the fiber to the penton base (Chroboczek *et al*, 1995), especially the conserved FNPVYP region in the tail (Arnberg *et al*, 1997). The C-terminus, or knob, is responsible for initial interaction with the cellular adenovirus receptor. After this initial binding, secondary binding between the capsid penton base and cell-surface integrins leads to internalization of viral particles in coated pits and endocytosis (Morgan *et al*, 1969; Svensson and Persson, 1984; Varga *et al*, 1991; Greber *et al*, 1993; Wickham *et al*, 1993). Integrins are αβ-heterodimers of which at least 19 α-subunits and 8 β-subunits have been identified (see http://nciarray.nci.nih.gov/cgi-bin/cards). The array of integrins expressed in cells is complex and will vary between cell types and cellular environment. Although the knob contains some conserved regions between serotypes, the knob proteins show a high degree of variability, indicating that different adenovirus receptors exist.

### Adenoviral serotypes

At present, six different subgroups of human adenoviruses have been proposed which in total encompass approximately 50 distinct adenovirus serotypes. Besides these human adenoviruses, many animal adenoviruses have been identified (e.g. Ishibashi and Yasue, 1984).
A serotype is defined on the basis of its immunological distinctiveness as determined by quantitative neutralization with animal antiserum (horse, rabbit). If neutralization shows a certain degree of cross-reaction between two viruses, distinctiveness of serotype is assumed if A) the hemagglutinins are unrelated, as shown by lack of cross-reaction on hemagglutination-inhibition, or B) substantial biophysical/biochemical differences in DNA exist (Francki *et al*, 1991). The serotypes identified last (42-49) were isolated for the first time from HIV infected patients (Hierholzer *et al*, 1988; Schnurr *et al*, 1993). For reasons not well understood, most of such immunocompromised patients shed adenoviruses that were never isolated from immunocompetent individuals (Hierholzer *et al*, 1988 and 1992; Khoo *et al*, 1995).

Besides differences towards the sensitivity against neutralizing antibodies of different adenovirus serotypes, adenoviruses in subgroup C such as Ad2 and Ad5 bind to different receptors as compared to adenoviruses from subgroup B such as Ad3 and Ad7 (Defer *et al*, 1990; Gall *et al*, 1996). Likewise, it was demonstrated that receptor specificity could be altered by exchanging the Ad3 knob protein with the Ad 5 knob protein, and vice versa (Krasnykh *et al*, 1996; Stevenson *et al*, 1995 and 1997). Serotypes 2, 4, 5 and 7 all have a natural affiliation towards lung epithelia and other respiratory tissues. In contrast, serotypes 40 and 41 have a natural affiliation towards the gastrointestinal tract. These serotypes differ in at least capsid proteins (penton-base, hexon), proteins responsible for cell binding (fiber protein), and proteins involved in adenovirus replication. It is unknown to what extend the capsid proteins determine the differences in tropism found between the serotypes. It may very well be that post-infection mechanisms determine cell-type specificity of adenoviruses. It has been shown that adenoviruses from subgroups A (Ad12 and Ad31), C (Ad2 and Ad5), D (Ad9 and Ad15), E (Ad4) and F (Ad41) are all able to bind labeled soluble CAR (sCAR) protein when immobilized on nitrocellulose. Furthermore, binding of adenoviruses with these serotypes to Ramos cells, that express high levels of CAR but lack integrins (Roelvink *et al*, 1996), could be efficiently blocked by addition of sCAR to these viruses prior to infection (Roelvink *et al*, 1998). However, the fact that (at least some) members of these subgroups are able to bind CAR does not exclude that these viruses have different infection efficiencies in various cell types. For example subgroup D viruses have relatively short fiber shafts compared to subgroup A and C viruses. It has been postulated that the tropism of subgroup D viruses is to a large extend determined by the penton base binding to integrins (Roelvink *et al*, 1996 and 1998). Another example is provided by Zabner *et al* (1998) who have tested 14 different serotypes on infection of human ciliated airway epithelia (CAE) and found that serotype 17 (subgroup D) was bound and internalized more efficiently then all other viruses, including other members of subgroup D. Similar experiments using serotypes from subgroup A-F in primary fetal rat cells showed that adenoviruses from subgroup A and B were bound and internalized inefficiently whereas viruses from subgroup D were most efficiently bound and internalized (Law *et al*, 1998). Also in this case viruses within one subgroup displayed different infection efficiencies. The importance of fiber binding for the improved infection of Ad17 in CAE was shown by Armentano *et al* (WO 98/22609A1) who made a recombinant Ad2/LacZ virus with a fiber gene from Ad17 and showed that the chimaeric virus infected CAE more efficient then Ad2/LacZ viruses with Ad2 fibers.

Thus despite their shared ability to bind CAR, differences in the length of the fiber, knob sequence and other capsid proteins e.g. penton base, of the different serotypes may determine the efficiency by which an adenovirus infects a certain target cell. Of interest in this respect is the ability of Ad2 and Ad5 fibers but not of Ad3 fibers to bind to fibronectin III and MHC class I derived peptides. This suggests that adenoviruses are able to use cellular receptors other than CAR (Hong *et al*, 1997). Serotypes 40 and 41 (subgroup F) are known to carry two fiber proteins differing in the length of the shaft. The long shafted 41L fiber is shown to bind CAR whereas the short shafted 41S is not capable of binding CAR (Roelvink *et al*, 1998). The receptor for the short fiber is not known.

### Adenoviral gene delivery vectors

Most adenoviral gene delivery vectors currently used in functional genomics, gene therapy or vaccination are derived from subgroup C adenoviruses Ad2 or Ad5. The vectors have at least a deletion in the E1 region that renders the recombinant virus replication defective. In this region, novel genetic information can then be introduced. It has been demonstrated extensively that recombinant adenoviruses, in particular serotype 5, are suitable for efficient transfer of genes *in vivo* to the liver, the airway epithelium and solid tumors in animal models and human xenografts in immunodeficient mice (Bout 1996, 1997; Blaese *et al*, 1995).
The use of adenoviral vectors in functional genomics includes building gene expression libraries and *in vitro* and *in vivo* gene validation with appropriate meaningful cell based assays or animal models for a particular human disease. Transfer and subsequent expression of a cDNA into a desired cell-type may lead to relevant phenotypic changes that may or may not confirm the role a particular cDNA plays in a particular disease. Alternatively such an exercise may lead to better insight into the validity of using a particular cDNA as a target for therapeutic intervention. In addition to sense copies of a gene or genes under investigation, antisense copies may be cloned into the adenoviral vector and used for validation studies.

Gene transfer vectors derived from adenoviruses (adenoviral vectors) have a number of features that make them particularly useful for gene transfer:
1) the biology of the adenoviruses is well characterized,
2) the adenovirus is not associated with severe human pathology,
3) the virus is extremely efficient in introducing its DNA into the host cell,
4) the virus can infect a wide variety of cells and has a broad host-range,
5) the virus can be produced at high titers in large quantities,
6) and the virus can be rendered replication defective by deletion of the early-region 1 (E1) of the viral genome (Brody and Crystal, 1994),
7) the vectors can be produced free of wildtype replicating adenovirus (WO 97/00326A1).

However, there are still a number of drawbacks associated with the use of adenoviral vectors:
1) adenoviruses, especially the well investigated serotypes Ad2 and Ad5, usually elicit an immune response by the host into which they are introduced,
2) the replication and other functions of the adenovirus, which are provided with the additional genetic material, are not always very well suited for the cells.
3) the serotypes Ad2 and Ad5 are not ideally suited for delivering additional genetic material to organs other than the liver. Delivery of vectors derived from Ad2 or Ad5 via the bloodstream leads to a significant delivery of these vectors to the cells of the liver. In therapies where other cell types then liver cells need to be transduced, some means of liver exclusion must be applied to prevent uptake of the vector by these cells. Current methods rely on the physical separation of the vector from the liver cells. This can be done by localizing the vector and/or the target organ via surgery, balloon angioplasty or direct injection into an organ via for instance needles. Liver exclusion is also being practiced by surgical targeting in which the vector is delivered to compartments in the body that are essentially isolated from the bloodstream. This prevents transport of the vector to the liver. Although these methods mostly succeed in avoiding gross delivery of the vector to the liver, most of the methods are crude and have still considerable leakage and/or have poor target tissue penetration characteristics. In some cases inadvertent delivery of the vector to liver cells can be toxic to the patient. For instance, delivery of a herpes simplex virus (HSV) thymidine kinase (TK) gene for the subsequent killing of dividing cancer cells through administration of ganciclovir, is quite dangerous when also a significant amount of liver cells are transduced by the vector. Significant delivery and subsequent expression of the HSV-TK gene to liver cells is associated with severe toxicity. Thus there is a discrete need for an inherently safe vector provided with the property of a reduced transduction efficiency of liver cells.
4) *In vitro* or *ex vivo* gene transfer for functional genomics using standard Ad2 or Ad5 adenoviral vectors can be very limited in particular cells of the hemopoietic system as well as cells of the vasculature, such as endothelial cells. In particular primary T-lymphocytes are difficult to transduce with adenoviral vectors, making vectors of this serotype difficult to use for *in vitro, in vivo* or *ex vivo* gene validation studies involving T-lymphocytes.

T-lymphocytes are primary targets in numerous gene therapy protocols. However, the use of subgroup C adenovirus serotypes 2 or 5 (Ad2 or Ad5) as a vector to transduce T-lymphocytes is hampered by its poor transduction efficiency for these cells. It has been shown by Wickham *et al* that poor T-cell transduction is due to lacking of both the primary Ad2-Ad5 receptor, used in attachment, and the secondary Ad receptor, which mediates entry of most adenovirus serotypes. Increasing adenoviral gene transfer into human T-lymphocytes has been achieved through the use of bispecific antibodies. Bispecific antibodies consisting of an antibody against adenoviral knob and an antibody against the pantropic marker CD3 have been used to transfer genes into resting T-lymphocytes (Wickham *et al*, 1997). The efficiencies that were achieved varied between 25 and 90%. The production of bispecific antibodies is done using chemical coupling methods such as succinimidyl-3-(2-pyridyldithiol)-propionate (SPDP) as a cross linking agent. Even though coupling of antibodies is technically feasible, these methods are prone to be difficult in terms of reproducibility. Furthermore, every time a transduction is done the adenoviral vector needs to be preincubated with the bi-specific antibodies to generate the targeted adenoviral vector, creating another variable in the procedure. Also important is the fact that anti-CD3 monoclonal antibodies activate the T-cell receptor activation signal (normally provided by antigen and antigen-presenting cells). The anti-CD3 monoclonal antibody most commonly used is OKT.sub.3, which is commercially available from Ortho Pharmaceuticals.

### SUMMARY OF THE INVENTION

In some particularly useful, but non-limiting aspects, the present invention provides functional genomics, gene therapy methods, compounds and medicines. Some other preferred, but non-limiting aspects and embodiments of the invention will be or become clear from the further description herein.

The present invention is particularly useful in applications where primary T-lymphocytes form the target cell type. The present invention relates to gene delivery vehicles provided with a tissue tropism for at least human T-lymphocytes.

Generally, it is an object of the invention to provide an improved means and method for providing a desired nucleotide sequence to a target cell, and in particular to a T-lymphocyte.

It is a particular object of the invention to provide a viral vector that has improved tropism for T-lymphocytes, e.g. such that said viral vector is more capable/efficient in providing a desired nucleotide sequence to a T-lymphocyte.

Generally, such a viral vector will be in the form of a viral particle comprising a viral coat (e.g. an envelope or a capsid) in which is contained/packaged a nucleic acid that encodes said desired nucleotide sequence, and usually also (at least part of) the viral genome.

One specific object of the invention is to provide an adenoviral vector that has increased tropism for T-lymphocytes, i.e. compared to the commonly used adenoviral vectors Ad2 and Ad5, and that still has all the advantages of (Ad2 or Ad5) adenoviral vectors.

Generally, the invention achieves this object by providing a (chimaeric) virus or virus particle that is suitable for use as a viral vector, and that has been provided with an altered/modified viral coat that confers upon said virus particle increased tropism for T-lymphocytes.

In particular, said virus particle may be provided with a coat comprising one or more coat proteins, at least one of which is altered, is modified and/or is replaced by a coat protein from another (type, subtype and/or serotype of) virus, so as to confer upon said virus particle increased tropism for T-lymphocytes, i.e. compared to the native virus particle.

In one particularly preferred embodiment of the invention, said at least one coat protein that is altered. modified and/or replaced (e.g. essentially fully or in part) will be a fiber (fibre). In this embodiment, the fiber may for instance be replaced by a fiber from another (type or subtype of) virus; or one or more parts of the amino acid sequence of the (native) fiber may be replaced by one or more parts of the amino acid sequence of a fiber from another (type, subtype and/or serotype of) virus.

In particular, in the practice of invention, the chimaeric virus particle will have been derived from a "first" virus type or subtype (for example from adenovirus Ad2 or Ad5) whereas the coat protein(s)/fiber that confer(s) upon said virus particle increased tropism for T-lymphocytes will have been derived (e.g. essentially fully and/or in part) from a different, "second" virus type or subtype (such as adenovirus Ad35 or Ad51).

The chimaeric viral particles of the invention may generally be prepared starting from a genetic construct that encodes the at least one desired nucleotide sequence and that further may encode (at least part of) the chimeric coat that provides the final viral particle with increased tropism for T-lymphocytes, as well as one or more further viral elements as further mentioned below. Generally, said preparation of the viral particles may be carried out by "packaging" said genetic construct in a suitable (packaging) cell to provide said chimeric viral particle, as will be further described hereinbelow. Accordingly, it is a further object of the invention is to provide such genetic constructs that may be packaged/used to provide a chimeric viral particle of the invention.

Thus, in a first aspect, the invention provides a chimaeric virus particle suitable for use as a vehicle for delivering at least one desired nucleotide sequence to a target cell, and in particular to a T-lymphocyte; which chimaeric virus particle comprises a (viral) coat, in which said coat:
a) is different from the coat that occurs in the native virus (particle), i.e. the virus (particle) from which the chimaeric virus particle has been derived;
b) provides said virus particle with increased tropism for a T-lymphocyte (e.g. compared to the native virus particle).

In particular, the invention provides a chimaeric virus particle suitable for use as a vehicle for delivering at least one desired nucleotide sequence to a target cell, and in particular to a T-lymphocyte; which chimaeric virus particle comprises a coat, which coat comprises one or more coat proteins, at least one of which:
a) is different from the (corresponding) coat protein that occurs in the native virus (particle);
b) provides said chimaeric virus particle with increased tropism for a T-lymphocyte (e.g. compared to the native virus particle).

The term "(viral) coat" as used herein (also) encompasses viral capsid(s) and/or viral envelope(s). Accordingly, the term "coat protein(s)" as used herein comprises any and all proteins that (together) constitute the viral coat, capsid and/or envelope, including but not limited to any fiber(s), penton(s), hexon(s). Usually, in the invention, the use of a viral particle that comprises a capsid - such as an adenovirus particle - will be preferred.

According to the invention, any one or more of the proteins which form the viral coat (e.g. capsid or envelope) may be modified, altered and/or replaced (e.g. essentially fully or in part) by one or more corresponding coat proteins derived from another virus, to provide the chimaeric virus particle with increased tropism for a T-lymphocyte as described herein.

Preferably, in the practice of the invention, one or more of the proteins which form the viral coat will be (essentially fully) replaced by one or more coat proteins derived from another (type, subtype or serotype of) virus to provide the chimaeric virus particle with increased tropism for a T-lymphocyte as described herein.

In particular, the at least one coat protein that is altered, modified and/or replaced (e.g. essentially fully and/or in part) so as to provide said chimaeric virus particle with increased tropism for a T-lymphocyte will be at least one fiber. Even more in particular, said at least one fiber will be replaced by a fiber derived from another (type, subtype or serotype of) virus so as to provide the chimaeric virus particle with increased tropism for a T-lymphocyte as described herein.

According to this latter embodiment, the invention provides a chimaeric virus particle suitable for use as a vehicle for delivering at least one desired nucleotide sequence to a target cell, and in particular to a T-lymphocyte; which chimaeric virus particle comprises a coat, which coat comprises at least one fiber, in which said fiber:
a) is different from the fiber that occurs in the native virus (particle);
b) provides said chimaeric virus particle with increased tropism for a T-lymphocyte (e.g. compared to the native virus particle).

In the invention, the (native) viral particle which is provided with the increased tropism for T-lymphocytes may be (derived from) any virus particle known per se, including but not limited to retrovirus, lentivirus, alphavirus, adeno-associated virus, or influenzavirus.

Preferably, said viral particle will be derived from an adenovirus.

When in said native viral particle, one or more coat protein(s) and/or a fiber - or any part(s) of such coat proteins or fiber - is replaced by one or more coat proteins and/or a fiber from another virus, said one or more coat proteins and/or said fiber - or said part(s) thereof - may derived from any one or more suitable (viral) sources, including but not limited to adenovirus, retrovirus, adeno associated virus (AAV), lentivirus, alphavirus or influenzavirus. Preferably, however, said coat protein(s) and/or fiber - or said part(s) thereof- will be derived from an adenovirus.

Accordingly, the coat protein(s) and/or fiber that are used to replace the coat protein(s) and/or fiber in the native viral particle may be derived from a virus (particle) that belongs to a different type or species than said native virus particle, for instance when a fiber or another coat protein of an adenovirus is build into a retrovirus or a lentivirus.

Also, the coat protein(s) and/or fiber that are used to replace the coat protein(s) and/or fiber in the native viral particle may be derived from virus (particle) that belongs to a different subgroup than the subgroup of the native virus particle, for instance when a fiber or another coat protein from an adenovirus of subgroup C is build into an adenovirus of subgroup B.

Also, the coat protein(s) and/or fiber that are used to replace the coat protein(s) and/or fiber in the native viral particle may be derived from a virus (particle) from a different subtype or serotype than the native viral particle, for instance when a fiber or another coat protein from an adenovirus from subtype Ad35 or Ad51 is build into an adenovirus particle of subtype Ad2 or Ad5 (in which Ad35/Ad51 and Ad2/Ad5, respectively, in this case also belong to different subgroups of adenovirus).

It is also possible to provide the native virus particle with increased tropism for T-lymphocytes by altering and/or modifying the at least one coat protein, e.g. by replacing one or more of the native coat proteins, and in particular by replacing the native fiber, with one or more coat proteins and/or a fiber the amino acid sequence of which has been altered and/or modified, such that the resulting altered/modified coat protein(s) or fiber provides the virus particle with increased tropism for T lymphocytes. alterations/modifications may for instance comprise substitution, addition, deletion and/or insertion of one or more amino acid residues, compared to the native amino acid sequence of the coat protein(s) and/or fiber.

For instance, it may be possible to use an analog, variant, mutant, part and/or fragment of a naturally occurring coat protein and/or fiber may be used, provided that such an analog, variant, mutant, part and/or fragment is different from the coat protein/fiber that occurs in the native virus (particle); and provided that such an analog, variant, mutant, part and/or fragment is capable of providing said chimaeric virus particle with increased tropism for a T-lymphocyte, i.e. compared to the native virus particle.

For instance, such an analog, etc., may have been derived from a virus (particle) of a different type/species, from a virus (particle) of a different subgroup, or from a virus (particle) of a different sub- or serotype. In addition, such an analog, etc., may have been derived from the coat protein and/or fiber that natively occurs in the virus (particle) from which the chimaeric virus particle has been derived.

It may also be possible to use a (native) coat protein, such as a (native) fiber, in which at least one part of the amino acid sequence of said coat protein has been replaced by at least one amino acid sequence derived from at least one other coat protein (and usually a corresponding coat protein) or fiber - i.e.from at least one other virus - so as to provide a chimaeric virus particle with increased tropism for T-lymphocytes.

One specific non-limiting example thereof may be the use of a fiber that is comprised of amino acid sequences derived from two or more different virusses - e.g. from two or more different adenovirus subtypes/serotypes, which may (also) include one or more sequences derived from the native adenovirus - which amino acid sequences together form the fiber that provides the viral vector with increased tropism for T-lymphocytes, as described above.

The chimaeric virus particle of the invention preferably has increased tropism for at least one (type of) T-lymphocyte, in particular for at least one (type of) T-lymphocyte derived from at least one species of animal, and more in particular for at least one (type of) T-lymphocyte derived from at least one species of mammal, including but not limited to T-lymphocytes derived from such mammals as human beings, rats, monkeys, horses and bovine.

In one particularly preferred embodiment, the chimaeric virus particle of the invention has increased tropism for at least one (type of) T-lymphocyte derived from a human being.

It should however be noted that, although the chimaeric virus particles of the invention have improved tropism for T-lymphocytes - and thus are most preferably used to provide the at least one desired nucleotide sequence to a T-lymphocyte - the chimaeric virus particle of the invention may in its broadest sense be used to deliver the desired nucleotide sequence to any desired target cell. These may include, but are not limited to, cells that are kept in vitro (e.g. in culture, for instance for functional genomics applications as described herein) or may be cells in vivo, e.g. a cell present in (a tissue or organ of) an animal, and in particular in a mammal including but not limited to a human being (e.g. for gene therapy applications).

These may include target cells such as, but not limited to T-lymphocytes (and/or subtypes thereof, including but not limited to CD3⁺ cells, CD3⁺CD4⁺CD8⁺, CD3⁺CD69⁺, CD69⁺, CD3⁺CD4⁺CD8⁻CD69⁺, CD3⁺CD4⁺CD8⁻CD69⁻, CD3⁺CD4⁻ CD8⁺CD69⁺ or CD3⁺CD4⁻CD8⁺CD69⁻ cells), B-lymphocytes, dendritic cells, and/or CD34⁺-cells. It may in particular include those cells which carry receptors and/or other proteins on their cell surface that are functionally equivalent to the receptors that are present on the cell surface of the T-lymphocytes and that are "recognized" by the coat protein/fiber used herein.

Preferably, however, the target cell is a T-lymphocyte, in particular a T-lymphocyte of a mammal, and more in particular a T-lymphocyte of a human being, which may again be present in vitro (e.g. in a culture of T-lymphocytes) or in vivo (e.g. in the body of such a animal, mammal and/or human being).

As mentioned above, the term "virus (particle)" as used herein indicates a particle that at least comprises a coat (meaning e.g. a capsid or an envelope) and at least one nucleic acid packaged within said coat, which nucleic acid encodes the nucleotide sequence to be provided to the target cell and preferably also (at least part of) the viral genome.

Preferably, the at least one nucleotide sequence to be provided to the target cell is present in the viral particle - i.e. in the nucleic acid packaged in said viral particle - in such a way that, upon infection of the target cell with the chimaeric virus particle, said at least one nucleotide sequence is transferred to the target cell, e.g. in a manner that allows for expression of said at least one nucleotide sequence in said target cell, and/or otherwise allows said at least one nucleotide sequence to provide and/or carry out its (intended) biological function in the target cell.

Optionally, a chimaeric virus particle of the invention may also include one or more further viral elements known per se, including but not limited to:
- one or more core proteins;
- one or more viral protease(s);
- one or more proteins necessary for the assembly of the coat and shut-down of host protein translation;
- one or more DNA binding proteins
- DNA- or RNA polymerases
- Reverse transcriptases.

In this respect, the chimaeric virus particle is preferably such that it is capable of providing the at least one desired nucleotide sequence to the target cell. Generally, this means that said chimaeric virus particle (and/or the nucleic acid packaged therein) should at least contain - i.e. besides the one or more proteins that form the coat - one or more, and preferably all, of the viral elements required for providing said at least one desired nucleotide sequence to the target cell.

Also, the chimaeric virus particle should preferably be such that it is incapable of independent replication. Such virus particles and their preparation will be known per se to the skilled person and/or will be as further described herein. For instance, for RCA-free adenovirus vectors and their production reference is generally made to International Application WO 97/00326.

The at least one desired nucleotide sequence may be any nucleotide sequence, either of known biological function, or of unknown biological function (e.g. when said function is to be determined, for instance as part of a functional genomics program). As such, the desired nucleotide sequence may encode an amino acid sequence (e.g. a protein such as an enzyme, a transporter, a kinase, phosphatase, a transcription factor or polypeptide) or an RNA sequence (e.g. mRNA, rRNA or tRNA); and/or may for instance be a cDNA, genomic DNA, previously cloned DNA, gene, EST, synthetic oligonucleotide, random sequence, antisense nucleic acid or genetic suppressor element.

In a particularly preferred embodiment of the invention, the chimaeric virus particle is or has been derived from an adenovirus (particle), i.e. to provide an adenoviral vector.

According to this embodiment, the invention thus provides a chimaeric virus particle, derived from an adenovirus (particle) and suitable for use as a vehicle for delivering at least one desired nucleotide sequence to a target cell, and in particular to a T-lymphocyte; which chimaeric virus particle comprises a capsid that:
a) is different from the capsid that occurs in the native adenovirus (particle);
b) provides said virus particle with increased tropism for a T-lymphocyte (e.g. compared to the native adenovirus particle).

In particular, the invention provides a chimaeric virus particle derived from an adenovirus (particle) and suitable for use as a vehicle for delivering at least one desired nucleotide sequence to a target cell, and in particular to a T-lymphocyte; which chimaeric virus particle comprises a capsid comprising one or more capsid proteins, at least one of which:
a) is different from the (corresponding) capsid protein that occurs in the native adenovirus (particle); and
b) provides said virus particle with increased tropism for a T-lymphocyte (e.g. compared to the native adenovirus particle).

Preferably, the at least one capsid protein that provides said chimaeric adenovirus particle with increased tropism for a T-lymphocyte is a fiber, a hexon, a penton, any combination thereof or a mutant derived thereof.

Most preferably, the at least one capsid protein that is altered,modified and/or replaced to provide said chimaeric virus particle with increased tropism for a T-lymphocyte, is a fiber. For instance, said fiber may be replaced by a fiber derived from another adenovirus (e.g. from another subgroup and/or another subtype or serotype).

Thus, in another aspect, the invention thus provides a chimaeric virus particle derived from an adenovirus (particle) and suitable for use as a vehicle for delivering at least one desired nucleotide sequence to a target cell, and in particular to a T-lymphocyte; which chimaeric virus particle comprises a capsid, which capsid comprises at least a fiber, in which said fiber:
a) is different from the fiber that occurs in the native adenovirus (particle);
b) provides said virus particle with increased tropism for a T-lymphocyte (e.g. compared to the native adenovirus particle).

Even more preferably, the at least one capsid protein or fiber that provides said chimaeric adenovirus particle with increased tropism for T-lymphocytes is also derived from an adenovirus. For instance, the at least one capsid protein and/or fiber may be derived from a "first" sub- or serotype of adenovirus, whereas the at least one capsid protein or fiber may have been derived from a different, "second" sub- or serotype of adenovirus; in which these "first" and "second" sub- or serotypes may belong to the same or different subgroups.

Inter alia for this purpose, a pertinent listing of human adenovirus subtypes is given hereinbelow. It should be noted that the invention in its broadest sense is not limited to the use of (adeno)virus particles and/or (adeno)viral coat proteins of human (adeno)virusses. For instance, also particles and/or coat proteins of non-human adenovirusses may be used:

### Human adenoviruses:

- Subgroup A: Adl2, Ad18, Ad31
- Subgroup B1: Ad3, Ad7, Ad16, Ad21, Ad51,
- Subgroup B2: Ad11, Ad14, Ad34, Ad35,
- Subgroup C: Ad1, Ad2, Ad5, Ad6,
- Subgroup D: Ad8, Ad9, Ad10, Adl3, Ad15, Ad17, Ad19, Ad20, Ad22-30, Ad32, Ad33, Ad36-39, Ad42-50
- Subgroup E: Ad4,
- Subgroup F: Ad40, Ad41.

Preferably, the first adenovirus (particle) - i.e. to which the capsid protein(s)/fiber is provided to afford a chimaeric adenovirus particle of the invention - is an adenovirus of subgroup C, and more preferably Ad2 or Ad5, with Ad5 being particularly preferred.

Also, preferably, the adenovirus from which the capsid protein/fiber is derived from is an adenovirus of subgroup B, and more preferably Ad35 or Ad51. With respect to adenovirus serotype "Ad51" as referred to herein, it should be noted that said serotype has been described in the article by de Jong et al., Journal of Clinical Microbiology, Dec. 1999, p. 3940-3945, as serotype *"Ad 50"* (which is also described in the de Jong reference as beloning to subgroup B1). In this resepct, it should further be noted that the adenovirus serotype referred to as *"Ad 51"* in the "De Jong"-reference (which is described as belonging to subgroup D) is herein referred to as adenovirus serotype "Ad 50".

Thus, in one preferred embodiment, the chimaeric adenovirus particle of the invention is an adenovirus particle of the sub-or serotype Ad5 at least provided with at least one capsid protein, and in particular the fiber, from an adenovirus of sub-or serotype Ad35 or Ad51.

In another preferred embodiment, the chimaeric adenovirus particle of the invention is an adenovirus particle of the sub- or serotype Ad2 at least provided with at least one capsid protein, and in particular the fiber, from an adenovirus of sub- or serotype Ad35 or Ad51.

Alternatively, an analog, variant, mutant, part and/or fragment of a naturally occuring adenoviral capsid protein and/or fiber may be used, which may again have been derived from an adenovirus of a different subgroup, subtype and/or serotype than the adenovirus (particle) from which the chimaeric adenovirus particle has been derived, provided that such an analog, variant, mutant, part and/or fragment is capable of providing said chimaeric adenovirus particle with increased tropism for a T-lymphocyte, i.e. compared to the native adenovirus particle.

In addition, such analogs, variants, mutants, parts and/or fragments may also have been derived from the capsid protein and/or fiber that natively occurs in the adenovirus (particle) from which the chimaeric adenovirus particle has been derived, again provided that such an analog, variant, mutant, part or fragment is different from the capsid protein that occurs in the native adenovirus (particle); and provided that such an analog, variant, mutant, part or fragment is capable of providing said chimaeric adenovirus particle with increased tropism for a T-lymphocyte compared to the native adenovirus particle.

Thus, according to one specific embodiment, the invention provides a chimaeric virus particle derived from a first sub- or serotype of adenovirus and suitable for use as a vehicle for delivering at least one desired nucleotide sequence to a target cell, and in particular to a T-lymphocyte; which chimaeric virus particle comprises a capsid comprising one or more capsid proteins, in which,
a) at least one capsid protein is derived from a sub- or serotype of adenovirus different from said first sub- or serotype; and in which
b)said at least one capsid protein provides said virus particle with increased tropism for a T-lymphocyte (e.g. compared to a native adenovirus particle of said first sub- or serotype).

More in particular, the invention provides a chimaeric virus particle derived from a first sub- or serotype of adenovirus and suitable for use as a vehicle for delivering at least one desired nucleotide sequence to a target cell, and in particular to a T-lymphocyte; which chimaeric virus particle comprises a capsid, which capsid comprises at least a fiber, in which said fiber:
a) is derived from a sub- or serotype of adenovirus different from said first sub-or serotype; and in which
b) said fiber provides said chimaeric virus particle with increased tropism for a T-lymphocyte (e.g. compared to a native adenovirus particle of said first subtype).

According to one particular embodiment, the invention provides a chimaeric virus particle derived from a first sub- or serotype of adenovirus and suitable for use as a vehicle for delivering at least one desired nucleotide sequence to a target cell, and in particular to a T-lymphocyte; which chimaeric virus particle comprises a capsid, which capsid comprises a fiber and one or more further capsid protein, in which:
a) at least said fiber is derived from a sub- or serotype of adenovirus different from said first sub- or serotype;
b) said fiber provides said chimaeric virus particle with increased tropism for a T-lymphocyte (e.g. compared to a native adenovirus particle of said first sub- or serotype);
and in which optionally:
c) at least one of the further capsid proteins is derived from the first sub- or serotype of adenovirus.

In this embodiment of the invention, besides the fiber, also one or more further capsid proteins may also have been derived from the "second" adenovirus, provided that at least one of the capsid proteins is (still) derived from the "first" adenovirus.

Also, more generally, in all the above aspects and embodiments of the invention, it is not excluded that, besides the coat protein(s)/fiber, the chimaeric virus particle of the invention in addition contains one or more further viral elements (e.g. as listed above) - and/or nucleotide sequences encoding such viral elements - that have been derived from the "second" virus (particle).

Preferably, in the invention, the "first" adenovirus (particle) - i.e. to which the capsid protein(s)/fiber is provided to afford a chimaeric adenovirus particle of the invention - is an adenovirus of subgroup C, and more preferably Ad2 or Ad5, with Ad5 being particularly preferred.

Also, preferably, the "second" adenovirus - i.e. from which the capsid protein/fiber is derived - is an adenovirus of subgroup B, and more preferably Ad35 or Ad51.

Thus, in one particularly preferred embodiment, the chimaeric adenovirus particle of the invention is an adenovirus particle of the sub- or serotype Ad5 at least provided with at least one capsid protein, and in particular the fiber, from an adenovirus of sub-or serotype Ad35 or Ad51.

In another particularly preferred embodiment, the chimaeric adenovirus particle of the invention is an adenovirus particle of the sub- or serotype Ad2 at least provided with at least one capsid protein, and in particular the fiber, from an adenovirus of sub-or serotype Ad35 or Ad51.

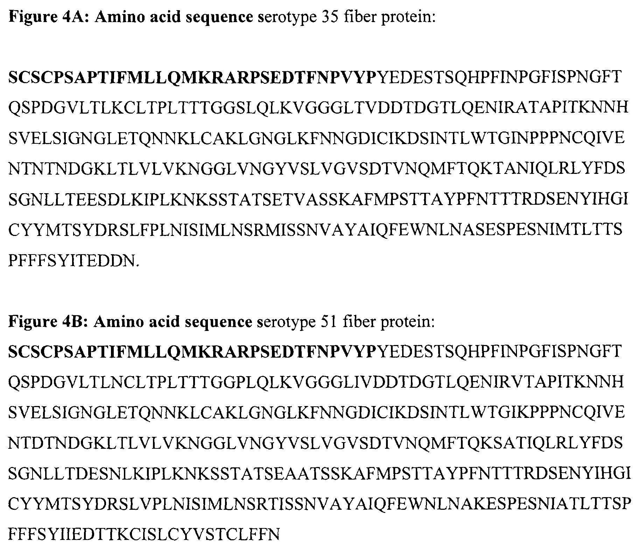

For instance, in the invention, the fiber protein of adenovirus Ad35 ( with the amino acid sequence shown in Figure 4A and SEQ ID NO:1) and/or the fiber protein of adenovirus Ad51 (with the amino acid sequence shown in Figure 4B and SEQ ID NO: 2) may be used to provide the "first" adenovirus (particle), and in particular Ad2 and/or Ad5, with increased tropism for T-lymphocytes.

Alternatively, a mutant, analog, variant, part or fragment of the amino acid sequence of SEQ ID NO:1 and/or SEQ ID NO:2 may be used, e.g. obtained by substitution, deletion, addition and/or insertion of one or more amino acid residues into or from the sequence of SEQ ID NO:1 and/or SEQ ID NO:2.

Preferably such a mutant, analog, variant, part or fragment still has a degree of amino acid homology with SEQ ID NO:1 and/or SEQ ID NO:2 of 50 %, preferably at least 70%, more preferably at least 80%, even more preferably at least 90%, with the sequence of SEQ ID NO:1 and/or SEQ ID NO:2; in which:
- the percentage amino acid homology is calculated by dividing the total number of amino acid residues that are identical to the amino acid residues on the corresponding amino acid position of SEQ ID NO: 1 (or SEQ ID NO:2 by the total number of amino acid residues of SEQ ID NO:1 (or SEQ ID NO:2); and multiplying by 100%
- each substitution, insertion, deletion or addition of an amino acid is considered an alteration at a single amino acid position;
- "conservative" amino acid substitutions may be taken into account.

Alternatively, the amount of amino acid homology may be determined using a suitable computer algorithm such as BLAST or PC-GENE at standard settings.

Also, instead of a such a synthetic mutant, analog, variant, part or fragment, also a naturally occurring analog or variant of the amino acid sequence of SEQ ID NO: 1 and/or SEQ ID NO:2 may be used, i.e. derived from a sub- or serotype of adenovirus different from Ad35 or Ad51.

Again, such a natural analog or variant preferably has a degree of amino acid homology (calculated as set out above) with SEQ ID NO: 1 and/or SEQ ID NO:2 of 50 %, preferably at least 70%, more preferably at least 80%, even more preferably at least 90%.

Also, preferably, the capsid protein/fiber is such that it provides the chimaeric virus particle of the invention, and in particular the chimaeric adenovirus particle of the invention, with a tropism for (at least one type of) T-lymphocytes (e.g. of at least one species of animal (mammal); and in particular from a human being), that is higher than the tropism of native Ad2 and/or Ad5 adenovirus.

More in particular, the capsid protein/fiber is such that it provides the chimaeric virus particle of the invention, and in particular the chimaeric adenovirus particle of the invention, with a tropism for (at least one type of) T-lymphocytes, as determined by the test described in Example 2 involving the introduction of eGFP into a T-lymphocyte, of at least 10%, preferably at least 30%, and in particular 40% or more. (By comparison, in said test, native Ad2 and Ad5 provide no more than 5 %).

In yet another aspect, the invention relates to the use of a chimaeric virus particle/viral vector as described above in providing at least one desired nucleotide sequence to a target cell.

The invention also relates to a genetic construct which may be used for providing a chimaeric virus particle/viral vector as described above. Usually, such a construct will be in the form of a nucleic acid (e.g. a DNA or RNA, and preferably a DNA) which encodes (at least part of) the genome of the chimaeric viral particle, and in particular (at least part of) the viral coat. Into said genetic construct may also be or have been inserted therein the one or more desired nucleotide sequences that are to be provided to the target cell.

The genetic construct is preferably such that it may be packaged in a suitable cell - such as a cell or a packaging cell line - so as to form a chimeric viral particle as described above, said particle at least comprising a viral coat with packaged therein a nucleotide sequence (e.g. encoding the viral genome and the at least one nucleotide sequence to be provided to the target cell).

When such a genetic construct encodes a chimeric adenovirus particle as described above, it may in particular be essentially as described in the international applications WO 97/00326 and/or PCT/NL/00367, which applications describe a range of E1-deleted adenovirus vectors that can be packaged and amplified using a suitable E1-complementing cell line, and optionally a suitable helper plasmid.

Generally, such a construct will at least contain, in an operable configuration:
- an expression cassette containing the one or more nucleotide sequences to be provided to the target cell;
- at least a left hand inverted terminal repeat;
- a packaging signal;
- and will essentially contain no E1 region sequences.

The constructs may be used to transfect/transduce a suitable cell or cell line, such as an E1-complementing cell line, so as to produce a chimeric viral particle of the invention. This viral particle may then be used to transfect the target cell, either in vitro or in vivo, e.g. so as to provide the intended nucleotide sequence to the target cell, e.g. for expression by/in the target cell.

All this may be carried out essentially as described in the international applications WO 97/00326 and/or PCT/NL99/00367. In this respect, it should be noted that these applications generally describe a broad range of different (types or classes of) genetic constructs encoding E1-deleted adenoviral vectors, such as constructs which besides the E1-deletion also do not contain/encode E2A, E2B, E3 and/or E4 region sequences. It is envisaged that the genetic constructs of the present invention encoding the chimeric (adeno)viral vectors may be in one or more of these forms.

Also, the international application PCT/NL99/00367 as well as the international application in the non-prepublished US provisional application 60191491, filed on March 21, 2000 and entitled *"Method for the preservation of virus particles"* describes (the use of) sets, collections and/or libraries of such constructs and/or libaries of adenoviral vectors obtained by packaging such constructs, as well as uses of such libraries, e.g. in high throughput screening.

Accordingly, it is envisaged that the chimeric viral particles/vectors - and/or the genetic constructs encoding such chimeric viral particles/vectors of the invention - may also be in the form of such a set, collection or library, i.e. containing at least 2, preferably at least 10 different viral particles - or constructs - in which the different viral vectors - or constructs - contained within said library may for instance differ in the nucleotide sequence to be provided to the target cell that they contain; and/or in their tropism for at least one T-lymphocytes (e.g. because each contruct/vector encodes/contains (a) different coat protein(s), leading to differences in such tropism.). Usually, a library - by which is meant a set or collection which covers the majority of, and up to essentially the entire, genome present in, and/or the majority of, and up to essentially all, cDNA's produced by a cell or organism of interest, will comprises at least 2 different sequences, e.g. between 5 and 1000 different sequences.

Such a set, collection or library may further be, and may be produced and/or used, essentially as described in PCT/NL99/00367 and/or in the non-prepublished US provisional application 60191491, filed on March 21, 2000 and entitled *"Method for the preservation of virus particles*", in that said set, collection or library may for instance be associated with a suitable carrier, such as a multi-well plate.

Usually, said genetic construct encoding the genome of the chimaeric virus particle will have been derived from the "first" virus as meant hereinabove (e.g. Ad2 or Ad5), in which the nucleotide sequences encoding the at least one capsid protein/fiber as meant hereinabove has been removed (or at least inactivated) and replaced with (at least) a nucleotide sequence encoding (at least) the capsid protein(s)/fiber derived from the "second" virus (particle), e.g. Ad35 or Ad51, and/or with a (usually synthetic) nucleotide sequence encoding an analog, mutant, variant, part or fragment as meant hereinabove.

The invention also relates to the use of the constructs described above in providing a chimaeric virus particle as described above, i.e. by packaging said construct in a suitable cell, and in particular a suitable packaging cell, so as to provide a chimaeric virus particle of the invention. Again, this may be carried out essentially as described in WO 97/00326 and/or PCT/NL99/00367. Again, this may also be carried out in a multi-well format and/or be automated.

Thus, as may be seen from the above and the further disclosure herein, the invention generally provides a gene delivery vehicle having been provided with at least a cell tropism for T-lymphocytes.

Said cell tropism is preferably being provided by a virus capsid, in which said capsid more preferably comprises protein fragments from at least two different viruses, of which virusses even more preferably at least one is an adenovirus, and in particular an adenovirus of subgroup B.

Even more in particular, said said subgroup B adenovirus may be adenovirus 35 or 51.

Also, in the above vehicles, at least one of said protein fragments comprises a tissue tropism determining fragment of a fiber protein derived from a subgroup B adenovirus, whereas the protein fragments not derived from an adenovirus of subgroup B are preferably derived from an adenovirus of subgroup C, preferably of adenovirus 5.

The vehicle of the invention also preferably comprises a nucleic acid derived from an adenovirus, which may in particular be derived from at least two different adenoviruses.

Preferably, said nucleic acid comprises at least one sequence encoding a fiber protein comprising at least a tissue or cell tropism determining fragment of a subgroup B adenovirus fiber protein, preferably of adenovirus 35 or 51.

Also, preferably, said adenovirus nucleic acid is modified such that the capacity of said adenovirus nucleic acid to replicate in a target cell has been reduced or disabled.

According to yet another aspect, said adenovirus nucleic acid may be modified such that the capacity of a host immune system to mount an immune response against adenovirus proteins encoded by said adenovirus nucleic acid has been reduced or disabled.

Also, preferably, the vehicle of the invention comprises a minimal adenoviral vector or an Ad/AAV chimaeric vector (http://patent.womplex.ibm.com/cgi-bin/viewpat.cmd/WO09932647A1).

The vehicle of the invention may further comprise at least one non-adenovirus nucleic acid, which is preferably a gene selected from the group of genes encoding RANLL/ODF, T-cell receptor genesand T-cell specific transcription factors. Also, said non-adenovirus nucleic acids may be a nucleic acid(s) may be taken from a gene collection or library. Also, when the nucleic acid forms part of such a collection or library, said nucleic acid(s) and/oe vehicle(s) may be arrayed and/or pooled.

The invention also relates to a cell for the production of a verhicle/vector as described above, said cell comprising means for the assembly of said vectors wherein said means includes a means for the production of an adenovirus fiber protein, wherein said fiber protein comprises at least a tissue tropism determining fragment of a subgroup B adenovirus fiber protein.

Preferably, said cell is, or is derived from, a PER.C6 cell (ECACC deposit number 96022940).

The vehicle of the invention may be used as a pharmaceutical, e.g. for the the treatment of cardiovascular disease, bone disorders, and/or a disease, treatable by transfer of a therapeutic nucleic acid to T-lymphocytes.

In yet another aspect, the invention relates to an adenovirus capsid with, or provided with, a tissue tropism for cells wherein said capsid preferably comprises proteins from at least two different adenoviruses and wherein at least a cell tropism determining fragment of a fiber protein is derived from a subgroup B adenovirus, preferably of adenovirus 35 or 51.

The above adenovirus may for instance be used for the delivery of nucleic acid to T-lymphocytes, and/or in a medicament - e.g. a gene therapy agent- for the treatment of a disease.

The invention also relates to one or more of the following constructs (further described below):
- pBr/Ad.BamRΔFib, at least comprising adenovirus 5 sequences 21562-31094 and 32794-35938;
- pBr/AdBamRfib51, at least comprising adenovirus 5 sequences 21562-31094 and 32794-35938, further comprising an adenovirus 51 gene encoding fiber protein;
- pBr/AdBamR.pac/fib51, at least comprising adenovirus 5 sequences 21562-31094 and 32794-35938, further comprising an adenovirus 51 gene encoding fiber protein, and further comprising a unique PacI-site in the proximity of the adenovirus 5 right terminal repeat, in the non-adenovirus sequence backbone of said construct;
- pWE/Ad.Af1IIrITRfib51, at least comprising adenovirus 5 sequences 3534-31094 and 32794-35938, further comprising an adenovirus 51 gene encoding fiber protein;
- pWE/Ad.Af1IIrITRDE2Afib51, at least comprising adenovirus 5 sequences 3534-22443, 24033-31094 and 32794-35938, further comprising an adenovirus 51 gene encoding fiber protein
- pBr/AdBamRfib35, at least comprising adenovirus 5 sequences 21562-31094 and 32794-35938, further comprising an adenovirus 35 gene encoding fiber protein;
- pBr/AdBamR.pac/fib35, at least comprising adenovirus 5 sequences 21562-31094 and 32794-35938, further comprising an adenovirus 35 gene encoding fiber protein, and further comprising a unique PacI-site in the proximity of the adenovirus 5 right terminal repeat, in the non-adenovirus sequence backbone of said construct;
- pWE/Ad.Af1IIrITRfib35, at least comprising adenovirus 5 sequences 3534-31094 and 32794-35938, further comprising an adenovirus 35 gene encoding fiber protein;
- pWE/Ad.Af1IIrITRDE2Afib35, at least comprising adenovirus 5 sequences 3534-22443, 24033-31094 and 32794-35938, further comprising an adenovirus 35 gene encoding fiber protein.

These constructs may optionally comprise at least one non-adenoviral nucleotide sequence, e.g. for delivery to a target cell as described herein.

The invention also relates to the use of a construct as generally described above, and/or to the use of one of the specific constructs described above, in or for the generation of a vehicle as mentioned above and/or an adenovirus capsid as mentioned above.

The invention also relates to the production of a vehicle as described above and/or of an adenovirus capsid as described above, which preferably at least comprises packaging a construct as described above in a suitable (packaging) cell, optionally using at least one suitable helper plasmid.

The invention also relates to the use of a vehicle as described above in or for the generation of a gene library.

The invention also relates to the use of (a nucleotide sequence encoding) a fiber protein of adenovirus 35 and/or 51 for (providing a vehicle for) the delivery of nucleic acid to T-lymphocytes, in which said vehicle is preferably as described above.

These and other aspects, embodiments, applications and advantages of the invention will also become clear and/or will be discussed in more detail in the further description given hereinbelow.

### Detailed description of the invention

It is an object of the current invention to provide materials and methods to overcome the limitations of adenoviral vectors mentioned above. In a broad sense, the invention provides new adenoviral viruses, derived in whole or in part from adenovirus serotypes different from Ad5. Specific genes of adenovirus serotypes with preferred characteristics may be combined in a chimaeric vector to give rise to a vector that is better suited for specific applications. Preferred characteristics include, but are not limited to, improved infection of a specific target cell, reduced infection of non-target cells, improved stability of the virus, reduced toxicity to target cells, reduced neutralization in humans or animals, reduced or increased CTL response in humans or animals, better and/or prolonged transgene expression, increased penetration capacity in tissues, improved yields in packaging cell lines, etc.

One aspect of the present invention facilitates the combination of the low immunogenicity of some adenoviruses with the characteristics of other adenoviruses that allow efficient gene delivery. Such characteristics may be a high specificity for certain host cells, a high rate of infection in certain host cells, low infection efficiency in non-target cells, etc.

The invention may thus provide chimaeric adenoviruses having the useful properties of at least two adenoviruses of different serotypes.

Typically, two or more requirements from the above non-exhaustive list are required to obtain an adenovirus capable of efficiently transferring genetic material to a host cell. Therefore the present invention provides adenovirus derived vectors which can be used as cassettes to insert different adenoviral genes from different adenovirus serotypes at the required sites. This way one can obtain a vector capable of producing a chimaeric adenovirus, whereby of course also a gene of interest can be inserted (for instance at the site of E1 of the original adenovirus). In this manner the chimaeric adenovirus to be produced can be adapted to the requirements and needs of certain hosts in need of gene therapy for certain disorders. To enable this virus production, a packaging cell will generally be needed in order to produce a sufficient amount of safe chimaeric adenoviruses.

In one of its aspects the present invention provides adenoviral vectors comprising at least a fragment of a fiber protein. Said fiber protein may be the native fiber protein of the adenoviral vector or may be derived from a serotype different from the serotype the adenoviral vector is based on. In the latter case the adenoviral vector according to the invention is a chimaeric adenovirus displaying at least a fragment of the fiber protein derived from subgroup B adenoviruses, which fragment comprising at least the receptor binding sequence. Typically such a virus will be produced using a vector (typically a plasmid, a cosmid or a baculoviral vector). Such vectors are also subject of the present invention. A preferred vector is a vector that can be used to make a chimaeric recombinant virus specifically adapted to the host to be treated and the disorder to be treated.

The present invention also provides a chimaeric adenovirus based on adenovirus type 5 but having at least a fragment of the fiber sequence from adenovirus type 35 or 51, whereby the fragment of the fiber of Ad35 or Ad51 comprises the fragment of the fiber protein that is involved in binding a host cell.

The present invention also provides chimaeric adenoviral vectors that show improved infection as compared to adenoviruses from other subgroups in specific host cells for example, but not limited to, CD3⁺ primary T-lymphocytes of human origin. An important feature of the present invention is the means to produce the chimaeric virus. Typically, one does not want an adenovirus batch to be administered to the host cell, which contains replication competent adenovirus. In general therefore it is desired to omit a number of genes (but at least one) from the adenoviral genome on the vector encoding the chimaeric virus and to supply these genes in the genome of the cell in which the vector is brought to produce chimaeric adenovirus. Such a cell is usually called a packaging cell. The invention thus also provides a packaging cell for producing a chimaeric adenovirus according to the invention, comprising in trans all elements necessary for adenovirus production not present on the adenoviral vector according to the invention. Typically vector and packaging cell have to be adapted to one another in that they have all the necessary elements, but that they do not have overlapping elements which lead to replication competent virus by recombination. Thus the invention also provides a kit of parts comprising a packaging cell according to the invention and a recombinant vector according to the invention whereby there is essentially no sequence overlap leading to recombination, resulting in the production of replication competent adenovirus, between said cell and said vector.

It is within the scope of the invention to insert more genes, or a functional part of these genes from the same or from other serotypes into the adenoviral vector replacing the corresponding native sequences. Thus for example replacement of (or a functional part of the) fiber sequences with corresponding sequences of other serotypes may be combined with, for example replacements of (or a functional part of) other capsid genes like penton base or hexon with corresponding sequences of said serotype or of other distinct serotypes. Persons skilled in the art understand that other combinations not limited to the said genes are possible and are within the scope of the invention.

In order to be able to precisely adapt the viral vector and provide the chimaeric virus with the desired properties at will, it is preferred that a library of adenoviral genes is provided whereby the genes to be exchanged are located on plasmid- or cosmid-based adenoviral constructs whereby the genes or the sequences to be exchanged are flanked by restriction sites. The preferred genes or sequences can be selected from the library and inserted in the adenoviral constructs that are used to generate the viruses. Typically, such a method comprises a number of restriction and ligation steps and transfection of a packaging cell. The adenoviral vector can be transfected in one piece, or as two or more overlapping fragments, whereby viruses are generated by homologous recombination. For example the adenoviral vector may be built up from two or more overlapping sequences for insertion or replacements of a gene of interest in for example the E1 region, for insertion or replacements in penton and/or hexon sequences, and for insertions or replacements into fiber sequences.

Of course it may not be necessary to make chimaeric adenoviruses that have complete proteins from different serotypes. It is well within the skill of the art to produce chimaeric proteins, for instance in the case of fiber proteins it is very well possible to have the base (i.e. "tail") of one serotype and the shaft and the knob from another serotype. In this manner it becomes possible to have the parts of the protein responsible for assembly of viral particles originate from one serotype, thereby enhancing the production of intact viral particles. Thus the invention also provides a chimaeric adenovirus according to the invention, wherein the hexon, penton, fiber and/or other capsid proteins are chimaeric proteins originating from different adenovirus serotypes. Besides generating chimaeric adenoviruses by swapping entire wild type capsid (protein) genes etc. or parts thereof, it is also within the scope of the present invention to insert capsid (protein) genes etc. carrying non-adenoviral sequences or mutations such as point mutations, deletions, insertions, etc. which can be easily screened for preferred characteristics such as temperature stability, assembly, anchoring, redirected infection, altered immune response etc. Again other chimaeric combinations can also be produced and are within the scope of the present invention.

In one embodiment this invention describes adenoviral vectors that are, amongst others, especially suited for gene delivery to human primary T-lymphocytes and T-cell derived cell-lines important for functional genomics based gene validation as well as for therapeutic interventions involving T-lymphocytes. The adenoviral vectors preferably are derived from subgroup B adenoviruses or contain at least a functional part of the fiber protein from an adenovirus from subgroup B comprising at least the cell-binding moiety of the fiber protein.

In a further preferred embodiment the adenoviral vectors are chimaeric vectors based on adenovirus type 5 and contain at least a functional part of the fiber protein from adenovirus type 51.

It is to be understood that in all embodiments the adenoviral vectors may be derived from the serotype having the desired properties or that the adenoviral vector is based on an adenovirus from one serotype and contains the sequences comprising the desired functions of another serotype, these sequences replacing the native sequences in the said serotype.

In another aspect this invention describes chimaeric adenoviruses and methods to generate these viruses that have an altered tropism different from that of adenovirus serotype 5. For example, viruses based on adenovirus serotype 5 but displaying any adenovirus fiber existing in nature. This chimaeric adenovirus serotype 5 is able to infect certain cell types more efficiently, or less efficiently *in vitro* and *in vivo* than the adenovirus serotype 5. Such cells include but are not limited to T-lymphocytes (and subtypes thereof as mentioned above) endothelial cells, smooth muscle cells, dendritic cells, hemopoietic stem cells, monocytic/macrophage cells, tumor cells, leukemic cells, skeletal muscle cells, synoviocytes, etc.

In another aspect the invention describes the construction and use of libraries consisting of distinct parts of adenovirus serotype 5 in which one or more genes or sequences have been replaced with DNA derived from alternative human or animal serotypes. This set of constructs, in total encompassing the complete adenovirus genome, allows for the construction of unique chimaeric adenoviruses customized for a certain disease, group of patients or even a single individual.

In all aspects of the invention the chimaeric adenoviruses may, or may not, contain deletions in the E1 region and insertions of heterologous genes linked to a promoter. Furthermore, chimaeric adenoviruses may, or may not, contain deletions in the E3 region and insertions of heterologous genes linked to a promoter. Furthermore, chimaeric adenoviruses may, or may not, contain deletions in the E2 and/or E4 region and insertions of heterologous genes linked to a promoter. In the latter case E2 and/or E4 complementing cell lines are required to generate recombinant adenoviruses. Alternatively these genes can be brought under transcriptional regulation such that these genes are only expressed when producing virus and not when using the vectors for functional genomics studies or therapeutic intervention. In fact any gene in the genome of the viral vector can be taken out and supplied in trans or be regulated. In the extreme situation, chimaeric viruses do not contain any adenoviral genes in their genome and are by definition minimal adenoviral vectors. In this case all adenoviral functions are supplied in trans using stable cell lines and/or transient expression of these genes. A method for producing minimal adenoviral vectors is described in WO97/00326 and is taken as reference herein.

In one embodiment the invention provides a gene delivery vehicle having been provided with at least a tissue tropism for T-lymphocytes. In a preferred embodiment of the invention said gene delivery vehicle is provided with a tissue tropism for at least T-lymphocytes using a fiber protein derived from a subgroup B adenovirus, preferably of adenovirus 35 or 51. In a preferred aspect of the invention said gene delivery vehicle comprises a virus capsid. Preferably said virus capsid comprises a virus capsid derived in whole or in part from an adenovirus of subgroup B, preferably from adenovirus 35 or 51, or it comprises proteins, or parts thereof, from an adenovirus of subgroup B, preferably of adenovirus 35 or 51. In a preferred embodiment of the invention said virus capsid comprises proteins, or fragments thereof, from at least two different viruses, preferably adenoviruses. In a preferred embodiment of this aspect of the invention at least one of said virus is an adenovirus of subgroup B, preferably adenovirus 35 or 51.

In a preferred embodiment of the invention said gene delivery vehicle comprises an adenovirus fiber protein or fragments thereof. Said fiber protein is preferably derived from an adenovirus of subgroup B, preferably of adenovirus 35 or 51. Said gene delivery vehicle may further comprise other fiber proteins, or fragments thereof, from other adenoviruses. Said gene delivery vehicle may, or may not, comprise other adenovirus proteins. Nucleic acid may be linked directly to fiber proteins, or fragments thereof, but may also be linked indirectly. Examples of indirect linkages include, but are not limited to, packaging of nucleic acid into adenovirus capsids or packaging of nucleic acid into liposomes, wherein a fiber protein, or a fragment thereof, is incorporated into an adenovirus capsid or linked to a liposome. Direct linkage of nucleic acid to a fiber protein, or a fragment thereof, may be performed when said fiber protein, or a fragment thereof, is not part of a complex or when said fiber protein, or a fragment thereof, is part of complex such as an adenovirus capsid.

In one embodiment of the invention is provided a gene delivery vehicle comprising an adenovirus fiber protein wherein said fiber protein comprises a tissue determining fragment of an adenovirus of subgroup B adenovirus preferably of adenovirus 35 or 51. Adenovirus fiber protein comprises three functional domains. One domain, the base, is responsible for anchoring the fiber to a penton base of the adenovirus capsid. Another domain, the knob, is responsible for receptor recognition whereas the shaft domain functions as a spacer separating the base from the knob. The different domains may also have other functions. For instance, the shaft is presumably also involved in target cell specificity. Each of the domains mentioned above may be used to define a fragment of a fiber. However, fragments may also be identified in another way. For instance the knob domain comprises of a receptor binding fragment and a shaft binding fragment. The base domain comprises of a penton base binding fragment and a shaft binding fragment. Moreover, the shaft comprises of repeated stretches of amino acids. Each of these repeated stretches may be a fragment. A tissue tropism determining fragment of a fiber protein may be a single fragment of a fiber protein or a combination of fragments of at least one fiber protein, wherein said tissue tropism determining fragment, either alone or in combination with a virus capsid, determines the efficiency with which a gene delivery vehicle can transduce a given cell or cell type, preferably but not necessarily in a positive way. With a tissue or cell tropism for T-lymphocytes is meant a tissue or cell tropism for cells having T-cell functions, preferably CD3 positive cells.

A tropism for a certain tissue may be provided by increasing the efficiency with which cells of said tissue are transduced, alternatively, a tropism for a certain tissue may be provided by decreasing the efficiency with which other cells than the cells of said tissue are transduced.

Fiber proteins possess tissue tropism determining properties. The most well described fragment of the fiber protein involved in tissue tropism is the knob domain. However, the shaft domain of the fiber protein also possesses tissue tropism determining properties. However, not all of the tissue tropism determining properties of an adenovirus capsid are incorporated into a fiber protein.
In a preferred embodiment of the invention, a fiber protein derived from a subgroup B adenovirus, preferably adenovirus 35 or 51, is combined with the non-fiber capsid proteins from an adenovirus of subgroup C, preferably of adenovirus 5.

In one aspect of the invention a gene delivery vehicle comprising a nucleic acid derived from an adenovirus is provided.

In a preferred embodiment of the invention, said adenovirus nucleic acid comprises at least one nucleic acid sequence encoding a fiber protein comprising at least a tissue tropism determining fragment of a subgroup B adenovirus fiber protein, preferably of adenovirus 35 or 51. In a preferred aspect said adenovirus comprises nucleic acid from at least two different adenoviruses. In a preferred aspect said adenovirus comprises nucleic acid from at least two different adenoviruses wherein at least one nucleic acid sequence encoding a fiber protein comprising at least a tissue tropism determining fragment of a subgroup B adenovirus fiber protein, preferably of adenovirus 35 or 51.
In a preferred embodiment of the invention, said adenovirus nucleic acid is modified such that the capacity of said adenovirus nucleic acid to replicate in a target cell has been reduced or disabled. This may be achieved through inactivating or deleting genes encoding early region 1 proteins.

An adenovirus nucleic acid may be altered further or instead of one or more of the alterations mentioned above, by inactivating or deleting genes encoding adenovirus late proteins such as but not-limited to, hexon, penton, fiber and/or protein IX.
In a preferred embodiment of the invention all genes encoding adenovirus proteins are deleted from said adenovirus nucleic acid, turning said nucleic acid into a minimal adenoviral vector.

In another preferred embodiment of the invention, a vector or a nucleic acid, which may be one and the same or not, according to the invention further comprises at least one non-adenovirus gene. Preferably, at least one of said non-adenovirus genes is selected from the group of genes encoding: T-cell relevant genes such as T-cell receptor genes, genes encoding T-cell specific secreted proteins and T-cell genes or anti T-cell genes involved in osteoclast differentiation such as RANKL/ODF. Or a cDNA from the library of genes such as described in WO 99/64582A2 can be the at least one non-adenovirus gene. Herefor arrayed or non-arrayed cDNA libraries can be build in adenoviral vectors, where the adenviral vector has tropism for at least T-lymphocytes. These libraries can be used in combination with T-lymphocyte specific assays such as T-lymphocyte proliferation and T-lymphocyte mediated cyto-toxicity.

In another aspect, the invention provides a cell for the production of a gene delivery vehicle provided with at least a tissue tropism for T-lymphocytes preferably of human origin. In another aspect, the invention provides a cell for the production of a gene delivery vehicle deprived of at least a tissue tropism for liver cells. In a preferred embodiment of the invention said cell is an adenovirus packaging cell, wherein an adenovirus nucleic acid is packaged into an adenovirus capsid. In one aspect of an adenovirus packaging cell of the invention all proteins required for the replication and packaging of an adenovirus nucleic acid, except for the proteins encoded by early region 1, are provided by genes incorporated in said adenovirus nucleic acid. The early region 1 encoded proteins in this aspect of the invention may be encoded by genes incorporated into the cells genomic DNA. In a preferred embodiment of the invention said cell is PER.C6 (ECACC deposit number 96022940). In general, when gene products required for the replication and packaging of adenovirus nucleic acid into adenovirus capsid are not provided by a adenovirus nucleic acid, they are provided by the packaging cell, either by transient transfection, or through stable transformation of said packaging cell. However, a gene product provided by the packaging cell may also be provided by a gene present on said adenovirus nucleic acid. For instance fiber protein may be provided by the packaging cell, for instance through transient transfection, and may be encoded by the adenovirus nucleic acid. This feature can among others be used to generate adenovirus capsids comprising of fiber proteins from two different viruses.

The gene delivery vehicles of the invention are useful for the treatment of diseases treatable by nucleic acid delivery toT-lymphocytes. A non-limiting example of the latter is for instance genetic disorders in which T-cells are involved such as SCID and AIDS.

The gene delivery vehicles of the invention may be used as a pharmaceutical for the treatment of said diseases. Alternatively, gene delivery vehicles of the invention may be used for the preparation of a medicament for the treatment of said diseases.
In one aspect the invention provides an adenovirus capsid with, or provided with, a tissue tropism for T-lymphocytes wherein said capsid preferably comprises proteins from at least two different adenoviruses and wherein at least a tissue tropism determining fragment of a fiber protein is derived from a subgroup B adenovirus, preferably of adenovirus 35 or 51. In another aspect the invention provides an adenovirus capsid deprived of a tissue tropism for liver cells wherein said capsid preferably comprises proteins from at least two different adenoviruses and wherein at least a tissue tropism determining fragment of a fiber protein is derived from a subgroup B adenovirus, preferably of adenovirus 35 or 51.

In another aspect of the invention is provided construct pBr/Ad.BamRΔFib, comprising adenovirus 5 sequences 21562-31094 and 32794-35938.

In another aspect of the invention is provided construct pBr/AdBamRfib35, comprising adenovirus 5 sequences 21562-31094 and 32794-35938, further comprising an adenovirus 35 gene encoding fiber protein.

In another aspect of the invention is provided construct pBr/AdBamR.pac/fib35, comprising adenovirus 5 sequences 21562-31094 and 32794-35938, further comprising an adenovirus 35 gene encoding fiber protein, and further comprising a unique PacI-site in the proximity of the adenovirus 5 right terminal repeat, in the non-adenovirus sequence backbone of said construct.

In another aspect of the invention is provided construct pWE/Ad.AflIIrITRfib35 comprising Ad5 sequence 3534-31094 and 32794-35938, further comprising an adenovirus 35 gene encoding fiber protein.

In another aspect of the invention is provided construct pWE/Ad.Af1IIrITRDE2Afib35 comprising Ad5 sequences 3534-22443 and 24033-31094 and 32794-35938, further comprising an adenovirus 35 gene encoding fiber protein.
In another aspect of the invention is provided construct pBr/AdBamRfib51, comprising adenovirus 5 sequences 21562-31094 and 32794-35938, further comprising an adenovirus 51 gene encoding fiber protein.

In another aspect of the invention is provided construct pBr/AdBamR.pac/fib51, comprising adenovirus 5 sequences 21562-31094 and 32794-35938, further comprising an adenovirus 51 gene encoding fiber protein, and further comprising a unique PacI-site in the proximity of the adenovirus 5 right terminal repeat, in the non-adenovirus sequence backbone of said construct.

In another aspect of the invention is provided construct pWE/Ad.Af1IIrITRfib51 comprising Ad5 sequence 3534-31094 and 32794-35938, further comprising an adenovirus 51 gene encoding fiber protein.

In another aspect of the invention is provided construct pWE/Ad.Af1IIrITRDE2Afib51 comprising Ad5 sequences 3534-22443 and 24033-31094 and 32794-35938, further comprising an adenovirus 51 gene encoding fiber protein. In the numbering of the sequences mentioned above, the number is depicted until and not until plus.

In a preferred embodiment of the invention, said constructs are used for the generation of a gene delivery vehicle or an adenovirus capsid with a tissue tropism for T-lymphocytes.

In another aspect the invention provides a library of adenoviral vectors, or gene delivery vehicles which may be one and the same or not, comprising a large selection of non-adenovirus nucleic acids. In another aspect of the invention, adenovirus genes encoding capsid proteins are used to generate a library of adenovirus capsids comprising of proteins derived from at least two different adenoviruses, said adenoviruses preferably being derived from two different serotypes, wherein preferably one serotype is an adenovirus of subgroup B. In a particularly preferred embodiment of the invention a library of adenovirus capsids is generated comprising proteins from at least two different adenoviruses and wherein at least a tissue tropism determining fragment of fiber protein is derived from an adenovirus of subgroup B, preferably of adenovirus 35 or 51.

A fiber protein of adenovirus 35 or 51 preferably comprises of the sequence given in figure 4. However within the scope of the present invention analogous sequences may be obtained through using codon degeneracy. Alternatively, amino-acid substitutions or insertions or deletions may be performed as long as the tissue tropism determining property is not significantly altered. Such amino-acid substitutions may be within the same polarity group or without.

In the following, the invention will be illustrated by means of the following non-limiting Experimental Part, as well as the non-limiting Figures, which show:
- Figure 1 (Example 1): Schematic drawing of the pBr/Ad.Bam-rITR construct.
- Figure 2 (Example 1): Schematic presentation of the strategy used to generate plasmid pBr/Ad.Bam-rITR fib in which the adenovirus type 5 fiber DNA is replaced by a short stretch containing an unique NsiI site.
- Figure 3 (Example 1): Schematic drawing of construct pBr/Ad.BamrITR fib.pac.
- Figure 4 (Example 1): Amino acid-sequences of the fiber proteins of adenovirus serotypes 35 and 51. Bold letters represent part of the tail of adenovirus type 5. At the end of the sequence the stopcodon of the fiber is presented by a dot.
- Figure 5 (Example 1): Schematic drawing of the adapter construct pAdApt/eGFP.
- Figure 6 (Example 1): Schematic presentation of the method to generate recombinant adenoviruses using two overlapping fragments. This system requires only one recombinational event. Early (E) and late regions (L) are indicated. L5 is the fiber coding sequence.
- Figure 7 (Example 2): Analysis of T-cells isolated from peripheral human blood. The total cell-population before isolation and the isolated T-cell population were unstained (resp. a en c) and stained for CD3(-PE) and CD45(-PerCP) expression (resp. b and d), followed by flow cytometry analysis to determine the percentage of CD3⁺CD45⁺-lymphocytes.
- Figure 8 (Example 2): Analysis of transduced T-lymphocytes. The T-cells were harvested and stained for CD3(-PE) expression, 48 hours after transduction, followed by flow cytometry analysis to determine the percentage of CD3⁺eGFP⁺ T-lymphocytes. Percentages given, are average percentages of eGFP⁺ cells in the CD3⁺ cell-population (average of two wells). Uninfected (a), Ad5\dE1.fib51.pAdApt/eGFP crude MOI 703 (b), Ad5\dE1.fib51.pAdApt/eGFP pure MOI 2500 (c).
- Figure 9 (Example 2): Flow cytometry results of transduced A549-, SupT1- and T-cells. A flow cytometer was used to determine the percentage of eGFP⁺ cells for A549 (a) and SupT1 (b). The T-cells were first stained with CD3-PE, and then the percentage of CD3⁺eGFP⁺ T-cells was determined (c). (Percentages given, are averages percentages of two wells.) Crude stands for crude lysates used as viruses, pure stands for purified viruses.

### EXPERIMENTAL PART.

### Example 1: Generation of adenovirus serotype 5 based viruses with chimearic fiber proteins.

The method described *infra* to generate recombinant adenoviruses by co-transfection of two, or more separate cloned adenovirus sequences. These cloned adenoviral sequences were subsequently used to remove specific adenovirus serotype 5 sequences in order to generate "template clones" which allow for the easy introduction of DNA sequences derived from other adenovirus serotypes. As an example of these template clones, the construction of plasmids enabling swapping of DNA encoding for fiber protein is given.

### I-1 Generation of adenovirus template clones lacking DNA encoding for fiber

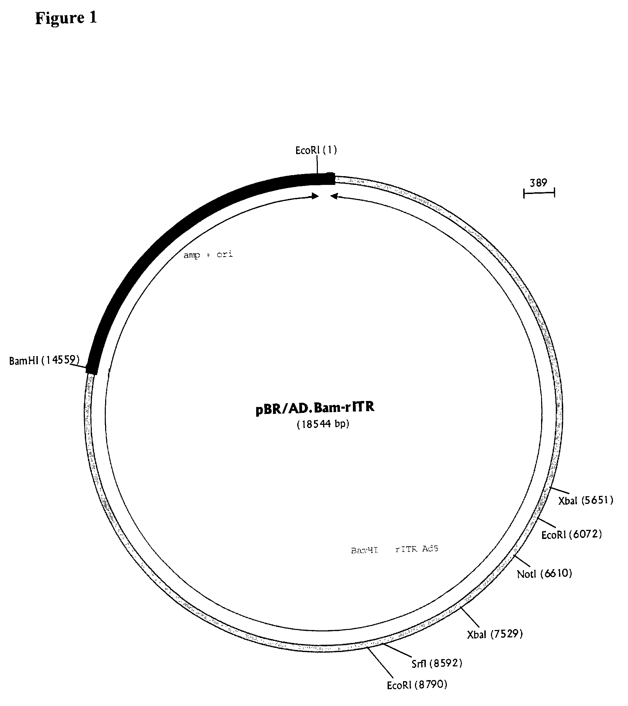

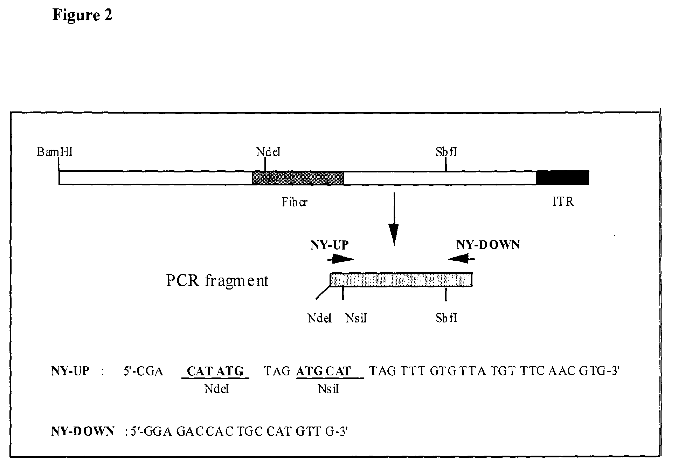

The fiber coding sequence of adenovirus serotype 5 is located between nucleotides 31042 and 32787. To remove the adenovirus serotype 5 DNA encoding fiber, we started with construct pBr/Ad.Bam-rITR (Figure 1; ECACC deposit p97082122). First a NdeI site was removed from this construct. For this purpose, pBr322 plasmid DNA was digested with NdeI after which protruding ends were filled using Klenow-enzym. This pBr322 plasmid was then re-ligated, digested with NdeI and transformed into *E*. *coli* DH5α. The obtained pBr/ΔNdeI plasmid was digested with Scal and SalI and the resulting 3198 bp vector fragment was ligated to the 15349 bp Scal-Sall fragment derived from pBr/Ad.BamrITR, resulting in plasmid pBr/Ad.BamrITRΔNdeI which hence contained a unique NdeI site. Next a PCR was performed with oligonucleotides "NY-up" and "NY-down". A schematic presentation of the strategy used to delete the fiber gene is shown in figure 2. During amplification, both a NdeI and a NsiI restriction site were introduced to facilitate cloning of the amplified fiber DNAs. Amplification consisted of
25 cycles of each 45 sec. at 94°C, 1 min. at 60°C and 45 sec. at 72°C. The PCR reaction contained 25 pmol of oligonucleotides NY-up or NY-down, 2mM dNTP, PCR buffer with 1.5 mM MgCl₂, and 1 unit of Elongase heat stable polymerase (Gibco, The Netherlands). One-tenth of the PCR product was run on an agarose gel which demonstrated that the expected DNA fragment of ± 2200 bp was amplified. This PCR fragment was subsequently purified using Geneclean kit system (Bio101 Inc.). Then, both the construct pBr/Ad.Bam-rITRΔNdeI as well as the PCR product were digested with restriction enzymes NdeI and SbfI. The PCR fragment was subsequently cloned using T4 ligase enzyme into the NdeI and SbfI digested pBr/Ad.Bam-rITRΔNdeI, generating pBr/Ad.BamRΔFib. This plasmid allows insertion of any PCR amplified fiber sequence through the unique NdeI and NsiI sites that are inserted in place of the removed fiber sequence. Viruses can be generated by a double homologous recombination in packaging cells described *infra* using an adapter plasmid, construct pBr/Ad.Af1II-EcoRI digested with Pad and EcoRI and a pBr/Ad.BamRΔFib construct in which heterologous fiber sequences have been inserted.

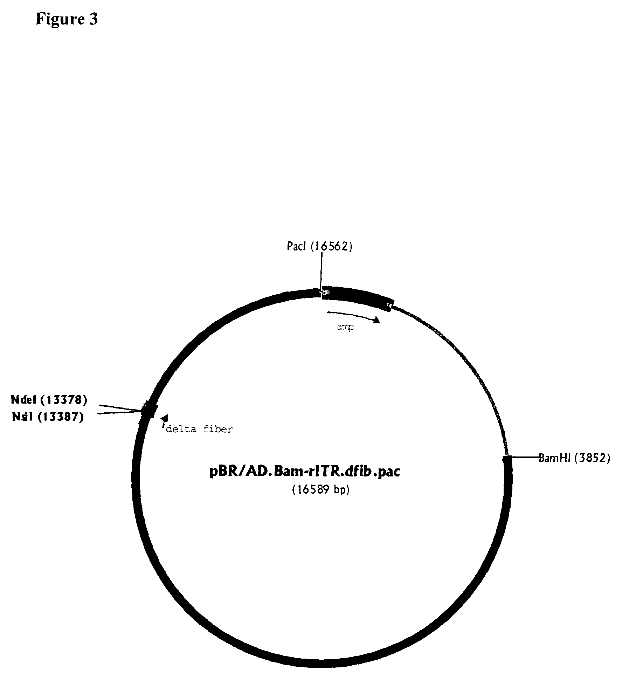

To increase the efficiency of virus generation, the construct pBr/Ad.BamRΔFib was modified to generate a PacI site flanking the right ITR. Hereto, pBr/Ad.BamRΔFib was digested with AvrII and the 5 kb adenofragment was isolated and introduced into vector pBr/Ad.Bam-rITR.pac#8 (ECACC deposit p97082121) replacing the corresponding AvrII fragment. The resulting construct was named pBr/Ad.BamRΔFib.pac (Figure 3). Once a heterologous fiber sequence is introduced in pBr/Ad.BamRΔFib.pac, the fiber modified right hand adenovirus clone may be introduced into a large cosmid clone pWE/Ad.Af1II-rITR. Such a large cosmid clone allows generation of adenovirus by only one homologous recombination making the process extremely efficient.

### I-2: Amplification of fiber sequences from adenovirus serotypes

To enable amplification of the DNAs encoding fiber protein derived from alternative serotypes degenerate oligonucleotides were synthesized. For this purpose, first known DNA sequences encoding for fiber proteins of alternative serotypes were aligned to identify conserved regions in both the tail-region as well as the knob-region of the fiber protein. From the alignment, (degenerate) oligonucleotides were synthesized (Table 1). Also shown in Table 1 is the combination of oligonucleotides used to amplify the DNA encoding fiber protein of a specific serotype. The amplification reaction (50 µl) contained
2 mM dNTPs, 25 pmol of each oligonucleotide, standard 1x PCR-buffer, 1.5 mM MgCl2, and 1 Unit Pwo heat stable polymerase (Boehringer) per reaction. The cycler program contained 20 cycles, each consisting of 30 sec. 94°C, 60 sec. 60-64°C and 120 sec. 72°C. One-tenth of the PCR product was run on an agarose gel which demonstrated that a DNA fragment was amplified. Of each different template, two independent PCR reactions were performed.

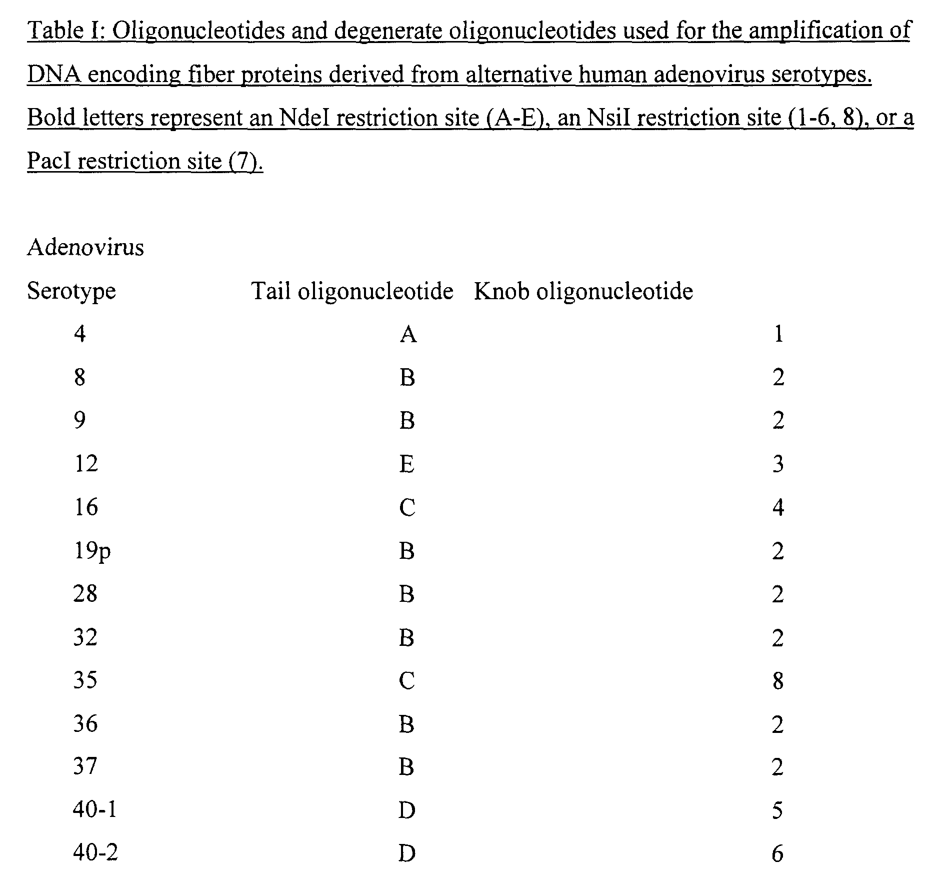

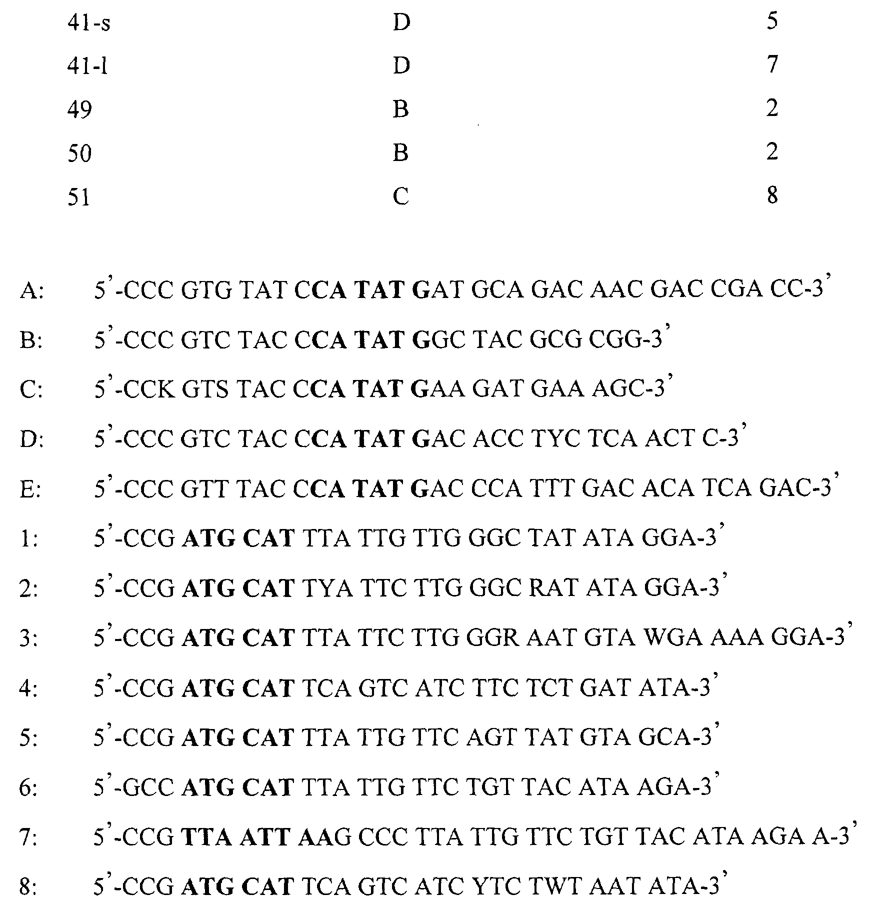

In the sequence listing, oligonucleotides A-E are given as SEQ ID's 3-7, and oligonucleotides 1-8 are given as SEQ ID's 8-15, respectively.

### I-3: Generation of fiber chimaeric adenoviral DNA constructs

Both amplified fiber DNAs as well as the vector (pBr/Ad.BamRΔFib) were digested with NdeI and NsiI. The digested DNAs was subsequently run on an agarose gel after which the fragments were isolated from the gel and purified using the Geneclean kit (Bio101 Inc). The PCR fragments were then cloned into the NdeI and NsiI sites of pBr/AdBamRΔFib, thus generating pBr/AdBamRFib35 and pBr/AdBamRFib51. The inserts generated by PCR were sequenced to confirm correct amplification. The obtained sequences of the different fiber genes are shown in Figure 4. From pBr/AdBamRFib35 and pBr/AdBamRFib51 PWE/Ad.Af1II-rITRfib35 and PWE/Ad.Af1II-rITRfib51 cosmids were generated as described above.

### I-4: Generation of recombinant adenovirus chimaeric for fiber protein

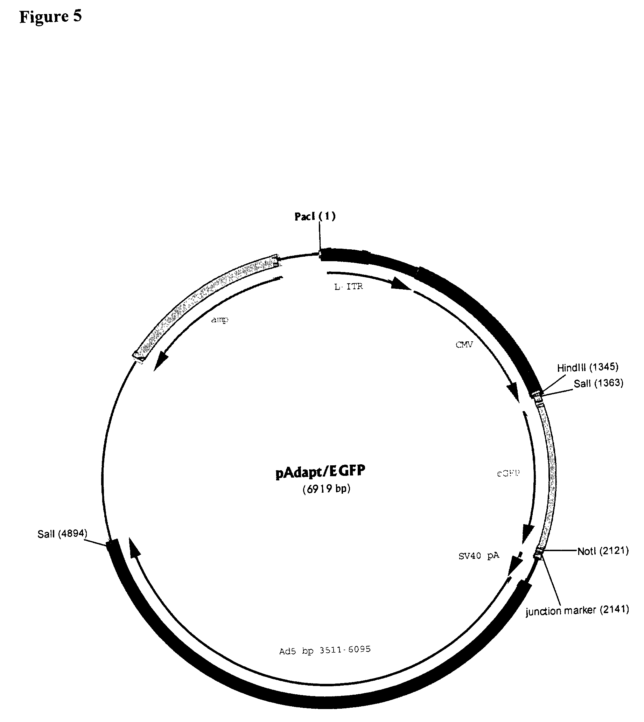

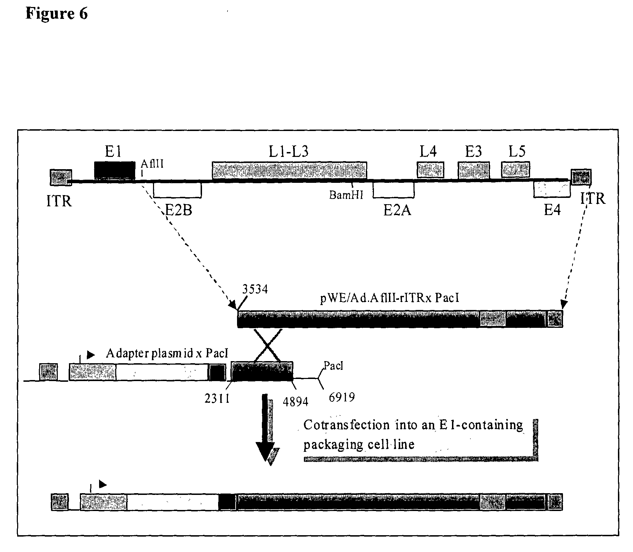

To generate recombinant Ad5 virus carrying the fiber of serotype 35 or 51, two contructs pAdApt/eGFP (Figure 5) and pWE/Ad.Af1II-rITR/Fib35 or pWE/Ad.Af1II-rITR/Fib51 were transfected into adenovirus producing cells (Figure 6). For transfection, 4 µg of pAdApt/eGFP linearized with Pad plus 4 µg of pWE/Ad.Af1II-rITR/Fib35 or pWE/Ad.Af1II-rITR/Fib51, also linearized with Pad, were diluted in serum free DMEM to 100 µl total volume. To this DNA suspension 100 µl 1x diluted lipofectamine (Gibco) was added. After 30 minutes at room temperature the DNA-lipofectamine complex solution was added to 2.5 ml of serum-free DMEM which was subsequently added to a T25 cm² tissue culture flask. This flask contained 2x10⁶ PER.C6 cells that were seeded 24-hours prior to transfection. Two hours later, the DNA-lipofectamine complex containing medium was diluted once by the addition of 2.5 ml DMEM supplemented with 9 mM MgCl₂ and 20% fetal calf serum. Again 24 hours later the medium was replaced by fresh DMEM supplemented with 9 mM MgCl₂ and 10% fetal calf serum. Cells were cultured for 6-8 days, subsequently harvested, and freeze/thawed 3 times. Cellular debri was removed by centrifugation for 5 minutes at 3000 rpm room temperature. Of the supernatant (12.5 ml) 3-5 ml was used to again infect PER.C6 cells in T80 m²tissue culture flasks. This re-infection results in full cytopathogenic effect (CPE) after 5-6 days, after which the adenovirus is harvested as described above. Again the virus is amplified. For this, 1 ml supernatant is used to infect PER.C6 cells (T175 cm² tissue culture flasks). This re-infection results in full cytopathogenic effect (CPE) after 4 days. Then the adenovirus is harvested, freeze/thawed
3 times and centrifuged for 5 minutes at 3000 rpm and room temperature. Supernatant is filtered through a 0,2/0,8 µm filter and aliquoted as crude lysates. The number of virusparticles per ml is determined by running the virus in a Quantitative Real-Time PCR. The virus titers that were found are: Ad5\dE1.fib35.pAdApt/eGFP 1.19 x 10¹⁰ vp/ml; Ad5\dE1.fib51.pAdApt/eGFP 2.81 x 10⁹ vp/ml.

### I-5: Production of fiber chimaeric adenoviruses

For amplifications a T175 cm² flasks with adherent perC6 cells with a confluency of ±70% are infected with 2,5 ml virus until this resulted in full cytopathogenic effect (CPE) within 3-5 days. The adenovirus was then harvested and freeze-thawed 3 times. Cellular debris was removed by centrifugation for 5 min at 3000 rpm and room temperature. Then 2.5 ml of the harvested virus was used to infect 4 T175 cm² 3-layer flasks that contained adherent PER.C6 cells with a confluency of ±70%. Three days after infection, the cells were harvested and pelleted by centrifugation for 5 min at 1500 rpm at room temperature. The chimaeric adenovirus present in the pelleted cells was subsequently extracted and purified using the following downstream processing protocol. The pellet was dissolved in 20 ml 10 mM NaPO₄ with 7% glycerol and frozen at -20°C. After thawing at 37°C, 2.25 ml deoxycholate (5% w/v) was added after which the solution was homogenized. The solution was subsequently incubated for 5 minutes at 37°C to completely crack the cells. After homogenizing the solution again, 750 µl 1M MgCl₂ and 150 µl DNase (10 mg/ ml; 10⁶ U/ml) was added. The solution is than homogenized and incubated for 15 minutes at 37°C. After 10 minutes the fluid was homogenized again. Cell debris was removed by centrifugation at 3000 rpm for 30 minutes at room temperature without the brake on. The supernatant was subsequently purified from proteins by loading on 20 ml of freon and centrifuged for 20 minutes at 2000 rpm without brake at room temperature. The upper fraction is then pipetted on a Tris/HCl (1M) buffered caesiumchloride blockgradient (range: 1.2 to 1.4 g/ml). Upon centrifugation at 21000 rpm for 2 hours at 10°C the virus was purified from remaining protein and cell-debris since the virusband will be positioned on the border of the 1.2 g/ml and the 1.4 g/ml caesiumchloride solution. The virus band is isolated after which a second purification using a Tris/HCl (1M) buffered continues gradient of 1.33 g/ml of caesiumchloride is performed. After virus loading on top of this gradient the virus is centrifuged for 17 hours at 55000 rpm at 10°C. Subsequently the virus band is isolated and 50 w/v% sucrose is added to the virus to a final concentration of 1 w/v%. Excess caesiumchloride is removed by three rounds of dialysis, each round comprising at least 1 hour. For dialysis the virus is transferred to dialysis slides (Slide-a-lyzer, cut off 10000 kDa, Pierce, USA). The buffers used for dialysis are PBS which are supplemented with an increasing concentration of sucrose (round 1 to 3: 30 ml, 60 ml, and 150 ml 50 w/v% sucrose / 1.5 liter cold PBS, all supplemented with 7.5 ml 2 w/v% CaMgCl₂). After dialysis, the virus is removed from the slide-a-lyzer after which it is aliquoted in portions of 50 µl upon which the virus is stored at -85°C. To determine the number of virusparticles per ml, 50 µl of the virus batch is run on a high pressure liquid chromatograph (HPLC) as described by Shamram *et al* (1997). The virus titers that were found are: Ad5\dE1.fib35.pAdApt/eGFP 1.3 x 10¹² vp/ ml; Ad5\dE1.fib51.pAdApt/eGFP 1.7 x 10¹² vp/ml.

### Example 2: Adenoviral transduction of human CD3⁺ T-lymphocytes with crude and purified vector preparations.

### II-1: Isolation and transduction of primary T-lymphocytes

To determine the transduction efficiency on human T-lymphocytes with chimaeric adenoviral vectors as described under example 1, CD3⁺ cells were isolated from peripheral human blood. First the mononuclear cells were isolated from the blood by spinning the blood through a Vacutainer Cell Preparation Tube with Sodium Heparine (Becton Dickinson) for 30 minutes at 3700 rpm, low acceleration and no brake. The cells were washed once with PBS. Then the mononuclear cells were subjected to cell affinity chromatography using the Pan T-cell isolation kit and the Macs system of Miltenyi Biotec for isolation of CD3⁺ cells. The isolated cells were treated with an ammonium-chloride solution of 155 mM for a period of 2 minutes on ice to eliminate the remaining erythrocytes.

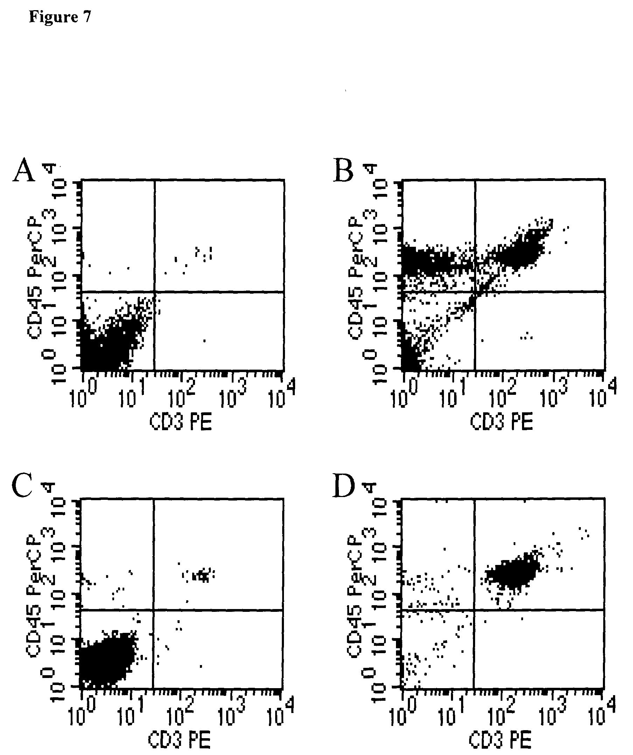

The percentage of CD3⁺ cells was determined by staining the isolated cells with CD3 antibodies labelled with PE (Becton and Dickinson) and CD45 antibodies labelled with PerCp (Becton and Dickinson) followed by flow cytometric analysis (Figure 7).

The CD3⁺ T-lymphocytes were then cultured in 24 well plates with 2 x 10⁵ cells per well using RPMI 1640 medium containing 10% heat inactivated FBS and incubation in a humidified CO₂ incubator set at 37°C and 10% CO₂. Transduction was performed in duplicate with 11 different adenoviral vectors carrying the eGFP transgene under the control of a CMV-promoter. The MOI that was used varied from 250 till
25000 VP/cell (Table II) in a total volume of 300 µls. Cells with virus were centrifuged for 5 min at 1500 rpm and incubated in a humidified CO₂ incubator set at 37°C and 10% CO₂. Control transductions included a human T-cell-line SupT1 and the ovarian cancer cell-line A549.

**Table 2 (Example 2):**

| Compilation of viruses used in example 2. MOIs are given in virusparticles (VP) /ml.(* means assumed MOI). Crude stands for crude lysates from adenoviral vector producing PER.C6 or PER.C6/E2A cells. Pure stands for viral vector purified through CsCl-banding. | | | |
|---|---|---|---|
| **Virus** | **MOI (Vp/cell) exp I** | **MOI(Vp/cell ) exp II** | **MOI(Vp/cell ) exp III** |
| Uninfected | 0 | 0 | 0 |
| Ad5\dE1.pIPspAdApt6/eGFP crude | 250 | | |
| Ad5\dE1.dE2A.pAdApt/eGFP crude | | 250, 2500 | 250, 2500 |
| Ad5\dE1.fib35.pAdApt/eGFP crude | 2975 | 250, 2500 | 250, 2500 |
| Ad5\dE1.fib51.pAdApt/eGFP crude | 703 | 250, 2500 | 250, 2500 |
| Ad5\dE1.fib40L.pAdApt/eGFP crude | 2500* | | |
| Ad5\dE1.fib45.pAdApt/eGFP crude | 2500* | | |
| Ad5\dE1.dE2A.pIPspAdApt6/empty crude | 1575 | 250, 2500 | 250, 2500 |
| Ad5\dE1.dE2A.pAdApt/eGFP pure | 2500 | 250, 2500 | 250, 2500 |
| Ad5\dE1.fib35.pAdApt/eGFP pure | 2500 | 2500, 25000 | 2500, 25000 |
| Ad5\dE1.fib51.pAdApt/eGFP pure | 2500 | 2500, 25000 | 2500, 25000 |
| Ad5\dE1.dE2A.pAdApt/empty pure | 2500 | 250, 2500 | 250, 2500 |

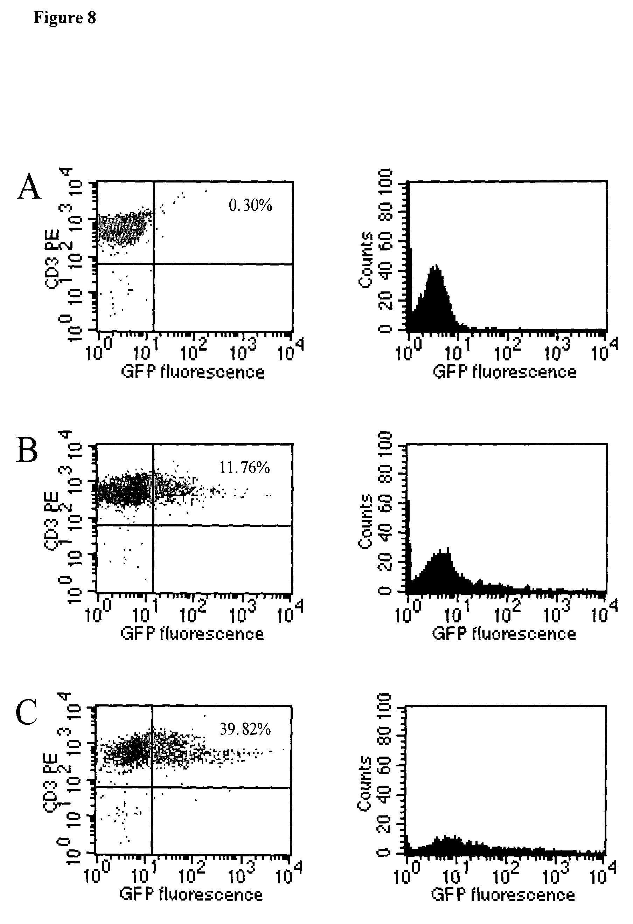

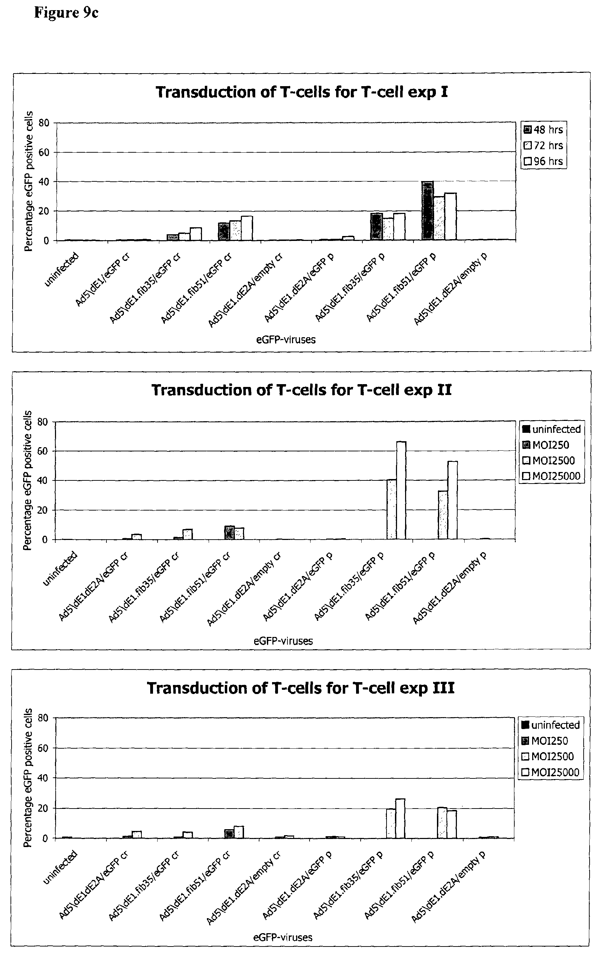

After 48, 72 or 96 hours (in experiment II and III after 48 hours) of incubation, the cells were transferred to tubes, washed once with PBS containing 0.5% BSA by centrifugation. Washing was followed by incubation with anti CD3-antibodies labelled with PE for 30 minutes on ice. The cells were then washed 2 times and resuspended in 200 µls of PBS with 0.5% BSA. The number of eGFP⁺CD3⁺ T-lymphocytes was then determined using a flow cytometer (Figure 8 and 9c).

### II-2: Transduction of human A549 cells

Infection of A549 cells (an human lung cell-line) was taken along as an infection control. Herefore A549 cells were seeded in 24 well plates with a density of 2 x 10⁵ cells/well and incubated in a humidified CO₂ incubator set at 37°C and 10% CO₂. Medium used was DMEM containing 10% heat inactivated FBS. Next day, they were transduced in duplo with 11 different adenoviral vectors carrying the eGFP transgene. The MOI that was used varied from 250 till 2500 VP/cell (Table II) in a total volume of 300 µls. Cells with virus were centrifuged for 5 min at 1500 rpm and incubated in a humidified CO₂ incubator set at 37°C and 10% CO₂.

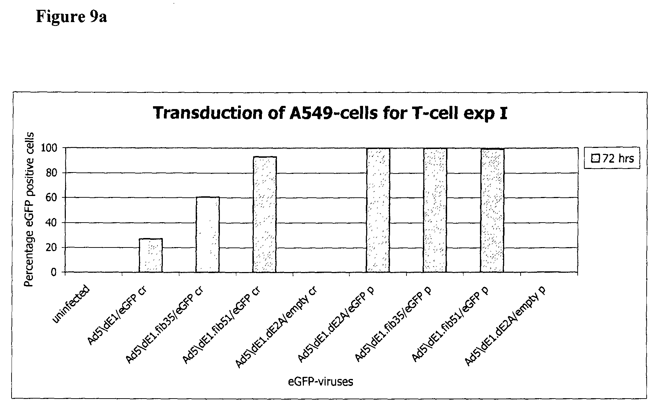

After 72 hours of incubation, the cells were transferred to tubes, washed with PBS containing 0.5% BSA by centrifugation, and resuspended in 200 µls of PBS with 0.5% BSA. The number of EGFP⁺ cells was then determined using a flow cytometer (Figure 9a).

### II-3: Transduction of human SupT1 cells

Infection of SupT1 cells (a human T-cell-line) was taken along as a second infection control. Herefore SupT1 cells were seeded in 24 well plates with a density of 2 x 10⁵ cells/well and incubated in a humidified CO₂ incubator set at 37°C and 10% CO₂. Medium used was RPMI 1640 containing 10% heat inactivated FBS. Next they were transduced in duplo with 11 different adenoviral vectors carrying the eGFP transgene. The MOI that was used varied from 250 till 25000 VP/cell (Table II) in a total volume of 300 µls. Cells with virus were centrifuged for 5 min at 1500 rpm and incubated in a humidified CO₂ incubator set at 37°C and 10% CO₂.

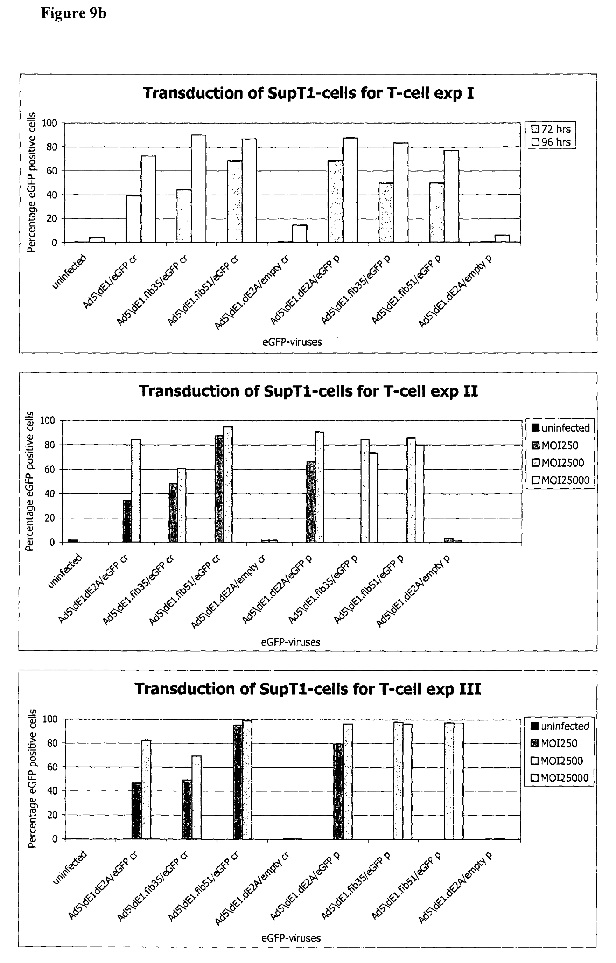

After 72 or 96 hours (in experiment II and III after 48 hours) of incubation, the cells were transferred to tubes, washed with PBS containing 0.5% BSA by centrifugation, and resuspended in 200 µls of PBS with 0.5% BSA. The number of EGFP⁺ cells was then determined using a flow cytometer (Figure 9b).

### II-4: Results

Clearly 2 out of 7 vectors tested as a PER.C6 or PER.C6.E2A adenoviral crude lysate show transduction levels varying from 3.78 to 16.43% eGFP positive CD3⁺T-lymphocytes (Figure 9). Better transduction levels were obtained using 2 out of the 4 purified PER.C6 or PER.C6.E2A adenoviral vectors. Levels are varying from 14.86 to 66.34% eGFP positive CD3⁺ T-lymphocytes. The adenoviral vectors with fibers of adenovirus serotype 35 and 51 are clearly positive for eGFP and thus, express the eGFP transgene. The adenoviral vector with the fiber of adenovirus serotype 5 (or fibers of serotype 40L and 45; data not shown) show undetectable levels of eGFP expression.
A549-cells and SupT1-cells are well transduced with all used viruses.

### Example 3: Transduction of activated T-lymphocytes and examining the possible effects of transduction on the activation status of naïve T-lymphocytes.

### III-1: Isolation and transduction of primary T-lymphocytes

To determine the transduction efficiency of activated T-cells and the effects of transduction itself on the activation status of naïve human T-lymphocytes, transductions were performed with chimaeric adenoviral vectors as described under example 1. After transduction, the CD3⁺ and or CD3⁺CD69⁺ cells were stained for expression of CD69, a marker for activated T-cells.

First the CD3⁺ T-lymphocytes were isolated from peripheral human blood, as described in example 2.

The percentage of CD3⁺ cells was determined by staining the isolated cells with CD3 antibodies labelled with PE (Becton and Dickinson) and CD45 antibodies labelled with PerCp (Becton and Dickinson) followed by flow cytometric analysis, to see if the correct cells were isolated.

The percentage of CD3⁺CD69⁺ cells pretransduction, was determined by staining the isolated cells with CD3 antibodies labelled with PE (Becton and Dickinson) and CD69 antibodies labelled with APC (Becton and Dickinson) followed by flow cytometric analysis. This is done to determine the activation status of the cells before transduction and after treatment with T-lymphocyte mitogens that activate T-lymphocytes, such as concanavalin A.

The CD3⁺ T-lymphocytes activated or not were then cultured and transduced as described in example 2. Control transductions included a human T-cell-line, SupT1. These transductions were performed in the same way as described in example 2.

After 48, 72 or 96 hours of incubation, the cells were transferred to tubes, washed once with PBS containing 0.5% BSA by centrifugation. Washing was followed by incubation with anti CD3-antibodies labelled with PE (Becton and Dickinson) and CD69-antibodies labelled with APC (Becton and Dickinson) for 30 minutes on ice. The cells were then washed 2 times and resuspended in 200 µls of PBS with 0.5% BSA. The number of eGFP⁺CD3⁺ and the number of eGFP⁺CD3⁺CD69⁺ T-lymphocytes was then determined using a flow cytometer.

### REFERENCES

- Abbas, A.K., Lichtman, A.H. and Pober, J.S. (1991) Cellular and Molecular Immunology. esp pag. 15-16
- Arnberg, N., Mei, Y. and Wadell, G. (1997) Fiber genes of adenoviruses with tropism for the eye and the genital tract. Virology 227: 239-244.
- Bergelson, J.M., Cunningham, J.A., Droguett, G., Kurt-Jones, E.A., Krithivas, A., Hong, J.S., Horwitz, M.S., Crowell, R.L. and Finberg, R.W. (1997) Isolation of a common receptor for coxsackie B virus and adenoviruses 2 and 5. Science 275: 1320-1323.
- Bout, A. (1997) Gene therapy, p. 167-182. In: D.J.A. Crommelin and R.D. Sindelar (ed.), Pharmaceutical Biotechnology, Harwood Academic Publishers.
- Bout, A. (1996) Prospects for human gene therapy. Eur. J. Drug Met. and Pharma. 2, 175-179.
- Blaese, M., Blankenstein, T., Brenner, M., Cohen-Hagenauer, O., Gansbacher, B., Russel, S., Sorrentino, B. and Velu, T. (1995) Cancer Gene Ther. 2: 291-297.
- Brody, S.L. and Crystal, R.G. (1994) Adenovirus mediated *in vivo* gene transfer. Ann. N. Y. Acad. Sci. 716: 90-101.
- Carter, A.J., Laird, J.R., Farb, A., Kufs, W., Wortham, D.C. and Virmani, R. (1994) Morphologic characteristics of lesion formation and time course of smooth muscle cell proliferation in a porcine proliferative restenosis model. J. Am. Coll. Cardiol. 24: 1398-1405.
- Castell, J.V., Hernandez, D., Gomez-Foix, A.M., Guillen, I, Donato, T. and Gomez-Lechon, M.J. (1997) Adenovirus-mediated gene transfer into human hepatocytes: analysis of the biochemical functionality of transduced cells. Gene Ther. 4(5): 455-464
- Chroboczek, J., Ruigrok, R.W.H., and Cusack, S. (1995) Adenovirus fiber, p. 163-200. In: W. Doerfler and P. Böhm (eds.), The molecular repertoire of adenoviruses I. Springer-Verlag, Berlin.
- Defer, C., Belin, M., Caillet-Boudin, M. and Boulanger, P. (1990) Human adenovirus-host cell interactions; comparative study with members of subgroup B and C. Journal of Virology 64(8): 3661-3673.
- Fallaux, F.J., Bout, A., van der Velde, I. *et al*. (1998) New helper cells and matched E1-deleted adenovirus vectors prevent generation of replication competent adenoviruses. Human Gene Therapy 9: 1909-1917.
- Francki, R.I.B., Fauquet, C.M., Knudson, D.L. and Brown, F. (1991) Classification and nomenclature of viruses. Fifth report of the international Committee on taxonomy of viruses. Arch. Virol. Suppl. 2: 140-144.
- Gall, J., Kass-Eisler, A., Leinwand, L. and Falck-Pedersen, E. (1996) Adenovirus type 5 and 7 capsid chimera: fiber replacement alters receptor tropism without affecting primary immune neutralization epitopes. Journal of Virology 70(4): 2116-2123.
- Greber, U.F., Willets, M., Webster, P., and Helenius, A. (1993) Stepwise dismanteling of adenovirus 2 during entry into cells. Cell 75: 477-486.
- Herz, J. and Gerard, R.D. (1993) Adenovirus-mediated transfer of low density lipoprotein receptor gene acutely accelerates cholesterol clearence in normal mice. Proc. Natl. Acad. Sci. U.S.A. 96: 2812-2816.
- Hierholzer, J.C. (1992) Adenovirus in the immunocompromised host. Clin. Microbiol Rev. 5: 262-274.
- Hierholzer, J.C., Wigand, R., Anderson, L.J., Adrian, T., and Gold, J.W.M. (1988) Adenoviruses from patients with AIDS: a plethora of serotypes and a description of five new serotypes of subgenus D (types 43-47). J. Infect. Dis. 158: 804-813.
- Hong, S.S., Karayan, L., Tournier, J., Curiel, D.T. and Boulanger, P.A. (1997) Adenovirus type 5 fiber knob binds to MHC class I (2 domain at the surface of human epithelial and B lymphoblastoid cells. EMBO J. 16: 2294-2306.
- Hsu, K.H., Lonberg-Holm, K., Alstein, B. and Crowell, R.L. (1988) A monoclonal antibody specific for the cellular receptor for the group B coxsackieviruses. J. Virol 62(5): 1647-1652.
- Huard, J., Lochmuller, H., Acsadi, G., Jani, A., Massie, B. and Karpati, G. (1995) The route of administration is a major determinant of the transduction efficiency of rat tissues by adenoviral recombinants. Gene Ther. 2:107-115.
- Imperiale, M.J., Akusjnarvi, G. and Leppard, K.N. (1995) Post-transcriptional control of adenovirus gene expression, P139-171. In: W. Doerfler and P. Böhm (eds.), The molecular repertoire of adenoviruses I. Springer-Verlag Berlin.
- Ishibashi, M. and Yasue, H. (1984) The adenoviruses, H.S. Ginsberg, ed., Plenum Press, Londen, New York. Chapter 12: 497-561.
- Jaffe, E.A., Nachman, R.L., Becker, C.G., Minick, C.R. (1973) Culture of endothelial cells derived from umbilical veins. Identification by morphologic and immunologic criteria. J. Clin. Invest. 52: 2745-2756.
- Kass-Eisler, A., Falck-Pederson, E., Elfenbein, D.H., Alvira, M., Buttrick, P.M. and Leinwand, L.A. (1994) The impact of developmental stage, route of administration and the immune system on adenovirus-mediated gene transfer. Gene Ther. 1:395-402.
- Khoo, S.H., Bailey, A.S., De Jong, J.C., and Mandal, B.K. (1995) Adenovirus infections in human immunodeficiency virus-positive patients: Clinical features and molecular epidemiology. J. Infect. Dis 172: 629-637.
- Kidd, A.H., Chroboczek, J., Cusack, S., and Ruigrok, R.W. (1993) Adenovirus type 40 virions contain two distinct fibers. Virology 192: 73-84.
- Krasnykh, V.N., Mikheeva, G.V., Douglas, J.T. and Curiel, D.T. (1996) Generation of recombinant adenovirus vectors with modified fibers for altering viral tropism. J. Virol. 70(10): 6839-6846.
- Krasnykh, V.N., Dmitriev, I., Mikheeva, G., Miller, C.R., Belousova, N. and Curiel, D.T. (1998) Characterization of an adenovirus vector containing a heterologous peptide epitope in the HI loop of the fiber knob. J. Virol. 72(3): 1844-1852.
- Law, L., Chillon, M., Bosch, A., Armentano, D., Welsh, M.J. and Davidson, B.L. (1998) Infection of primary CNS cells by different adenoviral serotypes: Searching for a more efficient vector. Abstract 1^{st} Annual Meeting American Society of Gene Therapy, Seattle, Washington.
- Leppard, K.N. (1997) E4 gene function in adenovirus, adenovirus vector and adeno-associated virus infections. J. Gen. Virol. 78: 2131-2138.
- Lloyd Jones, D.M. and Bloch, K.D. (1996) The vascular biology of nitric oxide and its role in atherogenesis. Annu. Rev. Med. 47: 365-375.
- Miltenyi, S., Muller, W., Weichel, W. and Radbruch, A. (1990) High gradient magnetic cell separation with MACS. Cytometry 11(2): 231-8.
- Morgan, C., Rozenkrantz, H.S., and Mednis, B. (1969) Structure and development of viruses as observed in the electron microscope.X. Entry and uncoating of adenovirus. J. Virol 4: 777-796.
- Roelvink, P.W., Kovesdi, I. and Wickham, T.J. (1996) Comparative analysis of adenovirus fiber-cell interaction: Adenovirus type 2 (Ad2) and Ad9 utilize the same cellular fiber receptor but use different binding stratagies for attachemnt. J. Virol. 70: 7614-7621.
- Roelvink, P.W., Lizonova, A., Lee, J.G.M., Li, Y., Bergelson, J.M., Finberg, R.W., Brough, D.E., Kovesdi, I. and Wickham, T.J. (1998) The coxsackie-adenovirus receptor protein can function as a cellular attachment protein for adenovirus serotypes from subgroups A, C, D, E, and F. J. Virol. 72: 7909-7915.
- Rogers, B.E., Douglas J.T., Ahlem, C., Buchsbaum, D.J., Frincke, J. and Curiel, D.T. (1997) Use of a novel cross-linking method to modify adenovirus tropism. Gene Ther. 4: 1387-1392.
- Schulick, A.H., Vassalli, G., Dunn, P.F., Dong, G., Rade, J.J., Zamarron, C. and Dichek, D.A. (1997) Established immunity precludes adenovirus-mediated gene transfer in rat carotid arteries.
- Schnurr, D and Dondero, M.E. (1993) Two new candidate adenovirus serotypes. Intervirol. 36: 79-83.
- Schwartz, R.S., Edwards, W.D., Huber, K.C., Antoniudes, L.C. Bailey, K.R., Camrud, A.R., Jorgenson, M.A. and Holmes, D.R. Jr. (1993) Coronary restenosis: Prospects for solution and new perspectives from a porcine model. Mayo Clin. Proc. 68: 54-62.
- Shi, Y., Pieniek, M., Fard, A., O'Brien, J., Mannion, J.D. and Zalewski, A. (1996) Adventitial remodelling after coronary arterial injury. Circulation 93: 340-348.
- Shabram, P.W., Giroux, D.D., Goudreau, A.M., Gregory, R.J., Horn, M.T., Huyghe, B.G., Liu, X., Nunnally, M.H., Sugarman, B.J. and Sutjipto, S. (1997) Analytical anion-exchange HPLC of recombinant type-5 adenoviral particles. Hum. Gene Ther. 8(4): 453-465.
- Signas, G., Akusjarvi, G., and Petterson, U. (1985) Adenovirus 3 fiberpolypeptide gene: Complications for the structure of the fiber protein. J. Virol. 53: 672-678.
- Stevenson, S.C., Rollence, M., White, B., Weaver, L. and McClelland, A., (1995) Human adenovirus serotypes 3 and 5 bind to two different cellular receptors via the fiber head domain. J. Virol 69(5): 2850-2857.
- Stevenson, S.C., Rollence, M., Marshall-Neff, J. and McClelland, A. (1997) Selective targeting of human cells by a chimaeric adenovirus vector containing a modified fiber protein. J. Virology 71(6): 4782-4790.
- Stouten, P.W.F., Sander, C., Ruigrok, R.W.H., and Cusack, S. (1992) New triple helical model for the shaft of the adenovirus fiber. J. Mol. Biol. 226: 1073-1084.
- Svensson, V. and Persson, R. (1984) Entry of adenovirus 2 into Hela cells. J. Virol.
   51: 687-694.
- Van der Vliet, P.C. (1995) Adenovirus DNA replication. In: W. Doerfler and P.
   Böhm (eds.), The molecular repertoire of adenoviruses II. Springer-Verlag, Berlin.
- Varga, M.J., Weibull, C., and Everitt, E. (1991) Infectious entry pathway of adenovirus type 2. J. Virol 65: 6061-6070.
- Varenne, O., Pislaru, S., Gillijns, H., Van Pelt, N., Gerard, R.D., Zoldhelyi, P., Van de Werf, F., Collen, D. and Janssens, S.P. (1998) Local adenovirus-mediated transfer of human endothelial nitric oxide synthetase reduces luminal narrowing after coronary angioplasty in pigs. Circulation 98: 919-926.
- Wadell, G. (1984) Molecular Epidemiology of human adenoviruses Curr. Top. Microbiol.Immunol. 110: 191-220.
- Wickham, T.J., Mathias, P., Cherish, D.A., and Nemerow, G.R. (1993) Integrins avb3 and avb5 promote adenovirus internalization but not virus attachment. Cell 73: 309-319.
- Wickham, T.J., Carrion, M.E. and Kovesdi, I. (1995) Targeting of adenovirus penton base to new receptors through replacement of its RGD motif with other receptor-specific peptide motifs. Gene Therapy 2: 750-756.
- Wickham, T.J., Segal, D.M., Roelvink, P.W., Carrion M.E., Lizonova, A., Lee, G-M., and Kovesdi, I. (1996) Targeted adenovirus gene transfer to endothelial and smooth muscle cells by using bispecific antibodies. J. Virol. 70(10): 6831-6838.
- Wickham, T.J., Lee, G-M., Titus, J.A., Sconocchia, G., Bakács, T., Kovesdi, I. and Segal, D.M. (1997) Targeted Adenovirus-mediated gene delivery to Tcells via CD3. J. Virol. 71: 7663-7669.
- Wijnberg, M.J., Quax, P.H.A., Nieuwenbroek, N.M.E., Verheijen, J.H. (1997) The migration of human smooth muscle cells *in vitro* is mediated by plasminogen activation and can be inhibited by al- pha(2)- macro globulin receptor associated protein. Thromb. and Haemostas. 78: 880-886.
- Wold, W.S., Tollefson, A.E. and Hermiston, T.W. (1995) E3 transcription unit of adenovirus. In: W. Doerfler and P. Böhm (eds.), The molecular repertoire of adenoviruses I. Springer-Verlag, Berlin.
- Zabner, J., Armentano, D., Chillon, M., Wadsworth, S.C. and Welsh, M.J. (1998) Type 17 fiber enhances gene transfer Abstract 1^{st} Annual Meeting American Society of Gene Therapy, Seattle, Washington.

## Claims

1. Chimaeric virus particle suitable for use as a vehicle for delivering at least one desired nucleotide sequence to a target cell, and in particular to a T-lymphocyte; which chimaeric virus particle comprises a viral coat, in which said coat:
a) is different from the coat that occurs in the native virus (particle), i.e. the virus (particle) from which the chimaeric virus particle has been derived;
b) provides said virus particle with increased tropism for a T-lymphocyte (e.g. compared to the native virus particle).

2. Chimaeric virus particle according to claim 1, which chimaeric virus particle comprises a coat, which coat comprises one or more coat proteins, at least one of which:
a) is different from the (corresponding) coat protein that occurs in the native virus (particle);
b) provides said chimaeric virus particle with increased tropism for a T-lymphocyte (e.g. compared to the native virus particle).

3. Chimaeric virus particle according to any of the preceding claims, in which one or more of the proteins which form the viral coat are essentially fully replaced by one or more coat proteins derived from another type, subtype or serotype of virus.

4. Chimaeric virus particle according to claim 3, in which said one or more coat proteins derived from another type, subtype or serotype of virus are derived from adenovirus, retrovirus, adeno associated virus (AAV), lentivirus, alphavirus or influenzavirus.

5. Chimaeric virus particle according to claim 3 and/or 4, in which said one or more coat proteins derived from another type, subtype or serotype of virus are derived from adenovirus.

6. Chimaeric virus particle according to any of the preceding claims, which chimaeric virus particle comprises a coat, which coat comprises at least one fiber, in which said fiber:
is different from the fiber that occurs in the native virus (particle);
provides said chimaeric virus particle with increased tropism for a T-lymphocyte (e.g. compared to the native virus particle).

7. Chimaeric virus particle according to claim 6, in which said fiber is essentially fully replaced by a fiber derived from another type, subtype or serotype of virus.

8. Chimaeric virus particle according to claim 7, in which said fiber derived from another type, subtype or serotype of virus is derived from adenovirus, retrovirus, adeno associated virus (AAV), lentivirus, alphavirus or influenzavirus.

9. Chimaeric virus particle according to claim 7 or 8, in which said fiber derived from another type, subtype or serotype of virus is derived from adenovirus.

10. Chimaeric virus particle according to any of claims 7-9, in which said fiber derived from another type, subtype or serotype of virus is derived from an adenovirus of subgroup B.

11. Chimaeric virus particle according to any of claims 7-10, in which said fiber derived from another type, subtype or serotype of virus is derived from an adenovirus Ad35 or Ad51.

12. Chimaeric virus particle according to any of the preceding claims, which is derived from a native viral particle chosen from adenovirus, retrovirus, lentivirus, alphavirus, adeno-associated virus, or influenzavirus.

13. Chimaeric virus particle according to any of the preceding claims, which comprises a capsid.

14. Chimaeric virus particle according to any of the preceding claims, which is derived from a native adenovirus.

15. Chimaeric virus particle according to any of the preceding claims, which is derived from a native adenovirus from subgroup C.

16. Chimaeric virus particle according to any of the preceding claims, which is derived from a native adenovirus chosen from Ad2 or Ad5.

17. Chimaeric virus particle according to any of the preceding claims, which has increased tropism for at least one (type of) T-lymphocyte from a human being.

18. Chimaeric virus particle according to any of the preceding claims, which is incapable of independent replication.

19. Chimaeric virus particle according to any of the preceding claims, comprising a nucleic acid contained/packaged with said viral coat, which nucleic acid encodes said at least one desired nucleotide sequence, and preferably also (at least part of) the viral genome.

20. Chimaeric virus particle according to any of the preceding claims, in which at least one the desired nucleotide sequence is a sequence of known biological function.

21. Chimaeric virus particle according to any claims 1-20, in which the at least one desired nucleotide sequence is a sequence of unknown biological function.

22. Chimaeric virus particle according to any of the preceding claims, in which the at least one desired nucleotide sequence is and/or encodes a cDNA, a genomic DNA, a previously cloned DNA, a gene, an EST, a synthetic oligonucleotide, a random sequence, an antisense nucleic acid or a genetic suppressor element.

23. Set, collection or library of virus particles, said set, collection or library comprising at least 2, preferably at least 10, different chimaeric virus particles according to any of claims 1-22.

24. Set, collection or library according to claim 23, associated with at least one carrier.

25. Set, collection or library according to claim 22 and/or 23, in a multi-well format.

26. Use of a chimaeric virus particle according to any of claims 1-22, and/or of a set, collection or library according to any of claims 23-25, in providing at least one desired nucleotide sequence to a target cell.

27. Use according to claim 26, in which the target cell is a T-lymphocyte (including but not limited to subtypes thereof), B-lymphocyte, dendritic cell, and/or a CD34+-cell, and/or a cell which carries on its cell surface a receptor and/or other protein that is functionally equivalent to the receptors that are present on the cell surface of the T-lymphocytes and that is recognized by the coat protein(s)/fiber present on the chimaeric virus particle used.

28. Use according to claim 26 and/or 27, in which the target cell is a T-lymphocyte.

29. Use according to any of claims 26-28, in which the target cell is a mammalian cell.

30. Use according to any of claims 26-29, in which the target cell is a human cell.

31. Use according to any of claims 26-30, in which the target cell is kept *in vitro*.

32. Use according to any of claims 26-31, in which the target cell is present *in vivo*.

33. Use according to any of claims, which is carried out in a high throughput setting.

34. Use according to any of claims, which is carried out in an automated fashion.

35. Genetic construct, which can be used for providing a chimaeric virus particle/viral vector according to any of claims 1-22, which construct is in the form of a nucleic acid which encodes (at least part of) the genome of the chimaeric virus particle, and in particular (at least part of) the viral coat of the chimaeric virus particle.

36. Genetic construct according to claim 35, which is such that it may be packaged in a suitable cell so as to form a chimaeric virus particle according to any of claims 1-22.

37. Genetic construct according to claim 35 and/or 36, containing, in an operable configuration:
an expression cassette containing the one or more nucleotide sequences to be provided to the target cell;
at least a left hand inverted terminal repeat;
a packaging signal.

38. Genetic construct according to any of claims 35-37, derived from an adenovirus.

39. Genetic contruct according to any of claims 35-38, which essentially contains no adenovirus E1 region sequences.

40. Set, collection or library of genetic constructs, said set, collection or library comprising at least 2, preferably at least 10, different genetic constructs according to any of claims 35-38

41. Set, collection or library according to claim 40, associated with at least one carrier.

42. Set, collection or library according to claim 40 or 41, in a multi-well format.

43. Use of a genetic construct according to any of claims 35-38, and/or of a set, collection or library according to any of claims 40-42, in producing at least one chimaeric virus particle, in particular at least one virus particle according to any of claims 1-22.

44. Use according to claim 43, in which said genetic construct is packaged in a suitable cell or cell line, and in particular a suitable packaging cell or cell line, so as to provide said chimaeric virus particle of the invention. optionally using a suitable helper plasmid.

45. Use according to claim 43 and/or 44, in which said construct is a construct according to claim 38 or 39, and in which said packaging cell or cell line is an E1-complementing cell or cell line.

46. Use according to claim 45, in which said packaging cell or cell line is (a cell of) the PER.C6 cell line(ECACC deposit number 96022940), or is (a cell of) a cell line derived from the PER.C6 cell line.

47. Use according to any of claims 43-46, which is carried out in a high throughput setting.

48. Use according to any of claims 43-47, which is carried out in an automated fashion.

49. Pharmaceutical composition, comprising at least one chimaeric virus particle according to any of claims 1-22, optionally associated with a pharmaceutically acceptable carrier, adjuvans or excipiens.


SEQUENCE LISTING

<110> Galapagos Genomics BV; IntroGene BV

<120> Gene delivery vectors provided with a tissue tropism
      for T-lymphocytes

<130> BO43694

<140>
<141>

<160> 15

<170> PatentIn Ver. 2.1

<210> 1
<211> 338
<212> PRT
<213> Adenovirus Ad35

<400> 1
Ser Cys Ser Cys Pro Ser Ala Pro Thr Ile Phe Met Leu Leu Gln Met
  1               5                  10                  15

Lys Arg Ala Arg Pro Ser Glu Asp Thr Phe Asn Pro Val Tyr Pro Tyr
             20                  25                  30

Glu Asp Glu Ser Thr Ser Gln His Pro Phe Ile Asn Pro Gly Phe Ile
         35                  40                  45

Ser Pro Asn Gly Phe Thr Gln Ser Pro Asp Gly Val Leu Thr Leu Lys
     50                  55                  60

Cys Leu Thr Pro Leu Thr Thr Thr Gly Gly Ser Leu Gln Leu Lys Val
 65                  70                  75                  80

Gly Gly Gly Leu Thr Val Asp Asp Thr Asp Gly Thr Leu Gln Glu Asn
                 85                  90                  95

Ile Arg Ala Thr Ala Pro Ile Thr Lys Asn Asn His Ser Val Glu Leu
            100                 105                 110

Ser Ile Gly Asn Gly Leu Glu Thr Gln Asn Asn Lys Leu Cys Ala Lys
        115                 120                 125

Leu Gly Asn Gly Leu Lys Phe Asn Asn Gly Asp Ile Cys Ile Lys Asp
    130                 135                 140

Ser Ile Asn Thr Leu Trp Thr Gly Ile Asn Pro Pro Pro Asn Cys Gln
145                 150                 155                 160

Ile Val Glu Asn Thr Asn Thr Asn Asp Gly Lys Leu Thr Leu Val Leu
                165                 170                 175

Val Lys Asn Gly Gly Leu Val Asn Gly Tyr Val Ser Leu Val Gly Val
            180                 185                 190

Ser Asp Thr Val Asn Gln Met Phe Thr Gln Lys Thr Ala Asn Ile Gln
        195                 200                 205

Leu Arg Leu Tyr Phe Asp Ser Ser Gly Asn Leu Leu Thr Glu Glu Ser
    210                 215                 220

Asp Leu Lys Ile Pro Leu Lys Asn Lys Ser Ser Thr Ala Thr Ser Glu
225                 230                 235                 240

Thr Val Ala Ser Ser Lys Ala Phe Met Pro Ser Thr Thr Ala Tyr Pro
                245                 250                 255

Phe Asn Thr Thr Thr Arg Asp Ser Glu Asn Tyr Ile His Gly Ile Cys
            260                 265                 270

Tyr Tyr Met Thr Ser Tyr Asp Arg Ser Leu Phe Pro Leu Asn Ile Ser
        275                 280                 285

Ile Met Leu Asn Ser Arg Met Ile Ser Ser Asn Val Ala Tyr Ala Ile
    290                 295                 300

Gln Phe Glu Trp Asn Leu Asn Ala Ser Glu Ser Pro Glu Ser Asn Ile
305                 310                 315                 320

Met Thr Leu Thr Thr Ser Pro Phe Phe Phe Ser Tyr Ile Thr Glu Asp
                325                 330                 335

Asp Asn

<210> 2
<211> 353
<212> PRT
<213> Adenovirus Ad51

<400> 2
Ser Cys Ser Cys Pro Ser Ala Pro Thr Ile Phe Met Leu Leu Gln Met
  1               5                  10                  15

Lys Arg Ala Arg Pro Ser Glu Asp Thr Phe Asn Pro Val Tyr Pro Tyr
             20                  25                  30

Glu Asp Glu Ser Thr Ser Gln His Pro Phe Ile Asn Pro Gly Phe Ile
         35                  40                  45

Ser Pro Asn Gly Phe Thr Gln Ser Pro Asp Gly Val Leu Thr Leu Asn
     50                  55                  60

Cys Leu Thr Pro Leu Thr Thr Thr Gly Gly Pro Leu Gln Leu Lys Val
 65                  70                  75                  80

Gly Gly Gly Leu Ile Val Asp Asp Thr Asp Gly Thr Leu Gln Glu Asn
                 85                  90                  95

Ile Arg Val Thr Ala Pro Ile Thr Lys Asn Asn His Ser Val Glu Leu
            100                 105                 110

Ser Ile Gly Asn Gly Leu Glu Thr Gln Asn Asn Lys Leu Cys Ala Lys
        115                 120                 125

Leu Gly Asn Gly Leu Lys Phe Asn Asn Gly Asp Ile Cys Ile Lys Asp
    130                 135                 140

Ser Ile Asn Thr Leu Trp Thr Gly Ile Lys Pro Pro Pro Asn Cys Gln
145                 150                 155                 160

Ile Val Glu Asn Thr Asp Thr Asn Asp Gly Lys Leu Thr Leu Val Leu
                165                 170                 175

Val Lys Asn Gly Gly Leu Val Asn Gly Tyr Val Ser Leu Val Gly Val
            180                 185                 190

Ser Asp Thr Val Asn Gln Met Phe Thr Gln Lys Ser Ala Thr Ile Gln
        195                 200                 205

Leu Arg Leu Tyr Phe Asp Ser Ser Gly Asn Leu Leu Thr Asp Glu Ser
    210                 215                 220

Asn Leu Lys Ile Pro Leu Lys Asn Lys Ser Ser Thr Ala Thr Ser Glu
225                 230                 235                 240

Ala Ala Thr Ser Ser Lys Ala Phe Met Pro Ser Thr Thr Ala Tyr Pro
                245                 250                 255

Phe Asn Thr Thr Thr Arg Asp Ser Glu Asn Tyr Ile His Gly Ile Cys
            260                 265                 270

Tyr Tyr Met Thr Ser Tyr Asp Arg Ser Leu Val Pro Leu Asn Ile Ser
        275                 280                 285

Ile Met Leu Asn Ser Arg Thr Ile Ser Ser Asn Val Ala Tyr Ala Ile
    290                 295                 300

Gln Phe Glu Trp Asn Leu Asn Ala Lys Glu Ser Pro Glu Ser Asn Ile
305                 310                 315                 320

Ala Thr Leu Thr Thr Ser Pro Phe Phe Phe Ser Tyr Ile Ile Glu Asp
                325                 330                 335

Thr Thr Lys Cys Ile Ser Leu Cys Tyr Val Ser Thr Cys Leu Phe Phe
            340                 345                 350

Asn

<210> 3
<211> 35
<212> DNA
<213> Artificial Sequence

<220>
<223> Description of Artificial Sequence:Synthetic
      oligonucleotide

<400> 3
cccgtgtatc catatgatgc agacaacgac cgacc                                35

<210> 4
<211> 27
<212> DNA

<213> Artificial Sequence

<220>
<223> Description of Artificial Sequence:Synthetic
      oligonucleotide

<400> 4
cccgtctacc catatggcta cgcgcgg                                          27


<210> 5
<211> 27
<212> DNA
<213> Artificial Sequence

<220>
<223> Description of Artificial Sequence:Synthetic
      oligonucleotide

<400> 5
cckgtstacc catatgaaga tgaaagc                                          27


<210> 6
<211> 31
<212> DNA
<213> Artificial Sequence

<220>
<223> Description of Artificial Sequence:Synthetic
      oligonucleotide

<400> 6
cccgtctacc catatgacac ctyctcaact c                                     31


<210> 7
<211> 36
<212> DNA
<213> Artificial Sequence

<220>
<223> Description of Artificial Sequence:Synthetic
      oligonucleotide

<400> 7
cccgtttacc catatgaccc atttgacaca tcagac                                36


<210> 8
<211> 30
<212> DNA
<213> Artificial Sequence

<220>
<223> Description of Artificial Sequence:Synthetic
      oligonucleotide

<400> 8
ccgatgcatt tattgttggg ctatatagga                                       30

<210> 9
<211> 30
<212> DNA
<213> Artificial Sequence

<220>
<223> Description of Artificial Sequence:Synthetic
      oligonucleotide

<400> 9
ccgatgcatt yattcttggg cratatagga                                        30


<210> 10
<211> 36
<212> DNA
<213> Artificial Sequence

<220>
<223> Description of Artificial Sequence:Synthetic
      oligonucleotide

<400> 10
ccgatgcatt tattcttggg raatgtawga aaagga                                 36


<210> 11
<211> 30
<212> DNA
<213> Artificial Sequence

<220>
<223> Description of Artificial Sequence:Synthetic
      oligonucleotide

<400> 11
ccgatgcatt cagtcatctt ctctgatata                                        30


<210> 12
<211> 30
<212> DNA
<213> Artificial Sequence

<220>
<223> Description of Artificial Sequence:Synthetic
      oligonucleotide

<400> 12
ccgatgcatt tattgttcag ttatgtagca                                        30


<210> 13
<211> 30
<212> DNA
<213> Artificial Sequence

<220>
<223> Description of Artificial Sequence:Synthetic
      oligonucleotide

<400> 13
gccatgcatt tattgttctg ttacataaga                                        30


<210> 14
<211> 37
<212> DNA
<213> Artificial Sequence

<220>
<223> Description of Artificial Sequence:Synthetic
      oligonucleotide

<400> 14
ccgttaatta agcccttatt gttctgttac ataagaa                                37


<210> 15
<211> 30
<212> DNA
<213> Artificial Sequence

<220>
<223> Description of Artificial Sequence:Synthetic
      oligonucleotide

<400> 15
ccgatgcatt cagtcatcyt ctwtaatata                                        30